# FASCICULE DE BREVET EUROPEEN

(11) **EP 0 646 574 B1**
(45) Date de publication et mention de la délivrance du brevet: **09.12.1998**
(21) Numéro de dépôt: 94402168.2
(22) Date de dépôt: 29.09.1994
(51) Int. Cl.: C07C 281/12, C07C 281/02, C07D 213/77

(54) **Dérivés d'indane cétoniques, leur procédé d'obtention, leur application thérapeutique**
Ketonderivate des Indans, ihre Herstellung und ihre therapeutische Anwendung
Ketonic indane derivatives, their preparation and therapeutic application thereof

(30) Priorité: 01.10.1993 FR 9311719
(43) Date de publication de la demande: 05.04.1995
(73) Titulaire: LABORATOIRE INNOTHERA Société Anonyme, 94111 Arcueil (FR)
(72) Inventeur: Desquand, Stéphanie, F-75015 Paris (FR); Finet, Michel, 32, boulevard Pasteur, F-94260 Fresnes (FR); Le Marquer, Florence, F-75005 Paris (FR); Tembo, Nornert Olivier, F-95800 Cergy Saint Christophe (FR); Torregrosa, Jean-Luc, F-93200 Saint Denis (FR); Yannic-Arnoult, Sylvie, F-91350 Grigny (FR)
(74) Mandataire: Dupuis-Latour, Dominique

(56) Documents cités:
- EP-A- 0 456 133
- EP-A- 0 566 445
- DE-A- 2 127 982
- CHEMICAL ABSTRACTS, vol. 108, no. 24, 1988, Columbus, Ohio, US; abstract no. 215426c, & J. INDIAN INST. SCI., vol.66, no.5, 1986 pages 317 - 323 K.G. REDDY ET AL

## Description

La présente invention concerne de nouveaux dérivés d'indane cétoniques, leur procédé d'obtention et leur application thérapeutique.

Il est décrit dans le "Journal of Organic Chemistry, vol. 38, N°12, 15 juin 1973, Easton US p. 2251-2", la transposition dans les systèmes d'indane 1,3-dione.

Le document Synthesis, N°3, mars 1973, Stuttgart, Allemagne Fédérale, pages 154-5, concerne l'amido-alkylation de dérivés de ninhydrines.

Le document Journal of Pharmaceutical Sciences, vol. 56, N°6, 6 juin 1967, Washington US, p. 775-6, décrit des thiosemicarbazones dérivées d'indane dione-1,3'.

Le document "Chemical Abstracts, vol. 72, N°11, 16 mars 1970", Columbus, Ohio, US, décrit de nouveaux dérivés de 1,3-indanedione thiosemicarbazone.

Le EP-A-0 566 445, publié le 20 octobre 1993 et donc opposable au seul titre de la nouveauté, décrit des dérivés d'indane-1,3-dione et d'indane-1,3-trione.

La présente invention a pour objet de nouveaux dérivés d'indane cétoniques tels qu'énoncés par la revendication 1.

La présente invention a également pour objet un procédé d'obtention de dérivés d'indane cétoniques tel qu'exposé dans la revendication 15. La sous-revendication 16 en vise un exemple particulier.

Les sous-revendications 2 à 13 visent divers cas particuliers de composés selon l'invention.

La présente invention comprend également les sels des composés salifiables mentionnés plus haut. Ces sels sont les sels d'addition d'acides minéraux tels que l'acide chlorhydrique, bromhydrique, sulfurique, phosphorique ou nitrique et les sels d'addition d'acides organiques tels que l'acide acétique, propionique, oxalique, citrique, maléique, fumarique, succinique et tartrique.

La présente invention a également pour objet l'utilisation des composés ci-dessus pour l'obtention d'un médicament destiné au traitement de l'insuffisance veineuse fonctionnelle, organique, des pathologies hémorroïdaires, des infections inflammatoires ostéo-articulaires, dermatologiques et cardiovasculaires, des états de chocs constitués par une chute importante de la pression artérielle plus particulièrement dans les états de chocs septiques.

L'invention vise également l'obtention comme produit intermédiaire du composé visé par la revendication 17. Les sous-revendications 22 et 23 en visent des exemples particuliers.

On indique ci-après la préparation des matières premières.

### exemple a

### 2-Pyridylaminocarbonylhydrazine

A 2.89g (17.14 mmoles) de 2-ethoxycarbonylaminopyridine, on ajoute à température ambiante 20 ml (411.5mmoles) d'une solution d'hydrazine à 98%, le mélange réactionnel est alors porté au reflux pendant 1h. Après complet refroidissement, le précipité obtenu est filtré sur verre fritté, lavé à l'éther puis au pentane pour fournir 2.6g de 2-Pyridylaminocarbonylhydrazine sous forme de cristaux blancs.
- **Rdt** : 100%
- **F** : 98°C
- **Rf** : 0.5 (CH₂Cl₂-MeOH, 98 : 2)
- **RMN du** ^{**1**}**H (60 MHz, DMSO d**_{**6**}**) : d (ppm)**
   8.90 (m, 1H, NH)
   8.25 (d, 1H, H-6, J5-6=5.5Hz)
   7.60 (m, 2H, H-3, H-4)
   6.75 (m, 1H, H-5)
   4.00 (ls, 3H, NH et NH₂)
- **IR (KBr)**
   υ NH₂ : 3345cm⁻¹, NH : 3205cm⁻¹, C=O : 1677cm⁻¹

### exemple b

### 3-Pyridylaminocarbonylhydrazine

A 32g (193 mmoles) de 3-ethoxycarbonylaminopyridine, on ajoute à température ambiante 37.5 ml (773mmoles) d'une solution d'hydrazine à 98%, le mélange réactionnel est alors porté au reflux pendant 30 minutes. Après complet refroidissement le milieu réactionnel est évaporé à sec puis chromatographié sur colonne de silice avec un mélange CH₂Cl₂/MeOH, 96/4 pour fournir 15g de 3-Pyridylaminocarbonylhydrazine sous forme de cristaux beiges.
- **Rdt :** 96%
- **F :**138°C
- **Rf :** 0.5 (CH₂Cl₂-MeOH, 91 : 9)
- **RMN du** ^{**1**}**H (60 MHz, DMSO d**_{**6**}**) : d (ppm)**
   8.8 (m, 2H, H-2, NH)
   8.0 (m, 2H, H-4, H-6)
   7.5 (ls, 1H, NH)
   7.2 (m, 1H, H-5)
   4.3 (ls, 2H, NH₂)
- IR (KBr)
   υ NH₂ : 3345cm⁻¹, NH : 3205cm⁻¹, C=O : 1677cm⁻¹

### exemple c

### 4-Pyridylaminocarbonylhydrazine

A 12 g (72 mmoles) de 2-éthoxycarbonylaminopyridine, on ajoute à température ambiante 40 ml (820 mmoles) d'une solution d'hydrazine à 98%, le mélange réactionnel est alors porté au reflux pendant 30 minutes. Après complet refroidissement, le précipité formé est essoré sur verre fritté rincé à l'eau glacée à l'éther et au pentane pour fournir 10,5 g de 4-pyridylaminocarbonylhydrazine sous forme de cristaux blancs.
- **Rdt**: 96%
- **F**: 98°C
- **Rf**: 0,2 (CH₂Cl₂-MeOH, 90:10)
- **RMN du** ^{**1**}**H (60 MHz, DMSO d**_{**6**}**) : δ (ppm)**
   9,0 (ls, 1H, NH)
   8,2 (m, 2H, H-3, H-5)
   7,6 (m, 3H, H-2, H-6, NH)
   4,3 (1s, 2H, NH₂)
- **JR (KBr)**
   υNH₂: cm⁻¹, NH: cm⁻¹, C=O : cm₋₁

L'invention est illustrée par les exemples 1 à 27, non limitatifs, ci-après :

### exemple 1

### 2.3-Dihydro-1.3-dioxo-2-[(2-nitrophenyl)aminocarbonylhydrazono]-1H-indène

A une solution de 6.21g (0.03 mole) de 2.3-dihydro-2.2-dihydroxy-1.3-dioxo-1H-indène dans 50ml d'éthanol, on ajoute 5.34g (0.03 mole) de (2-nitrophényl) aminocarbonylhydrazine et 0.5ml d'acide chlorhydrique concentré.

Le mélange réactionnel est porté à 80°C pendant 30 minutes et le précipité orange qui apparait est essoré, lavé à l'eau, séché puis purifié sur colonne Buchi moyenne pression sur silice (éluant : CH₂Cl₂).Les cristaux jaunes obtenus après évaporation du solvant sont décolorés au charbon activé et recritallisés dans du dichlorométhane.

Cristaux jaunes
**Rdt** : 80%
**F** : >260°C
**Rf** : 0.34 (CH₂Cl₂)
**RMN du** ^{**1**}**H (270 MHz, DMSO d**_{**6**}**): d (ppm)**
   12.37 (ls, 1H, NH)
   11.92 (ls, 1H, NH)
   8.13 (d, 1H, H-3', JH3'-4 '=8.41Hz)
   7.99 (m, 5H, H-4, H-5, H-6, H-7, H-6')
   7.83 (m, 1H, H-5')
   7.38 (dd, 1H, H-4', J H3'-4' = 8.41Hz, JH4'-5'=7.71Hz)
**RMN du** ^{**13**}**C (270 MHz, DMSO d**_{**6**}**): d (ppm)**
   186.22 (C=O), 185.61 (C=O), 151.25 (C=O), 140.13 (C-2, C-3a, C-7a), 136.40 (C-5, C-6), 135.85 (C-2'), 134.94 (C-1') , 132.12 (C-3') , 125.51 (C-6'), 124.71 (C-5'), 123.96 (C-4'), 123.45 (C-4, C-7),
**SM (I.E)** : m/z 338 (M^{+.})
-**IR (KBr)**
   υ NH : 3288, 3198 cm⁻¹, C=O : 1727 et 1687 cm^{-1.}

### exemple 2

### 2.3-Dihydro-1.3-dioxo-2-[(3-nitrophenyl)aminocarbonylhydrazono]-1H-indène

A une solution de 6.21g (0.03 mole) de 2.3-dihydro-2.2-dihydroxy-1.3-dioxo-1H-indène dans 50ml d'éthanol,on ajoute 5.34g (0.03 mole) de (3-nitrophényl) aminocarbonylhydrazine et 0.5ml d'acide chlorhydrique concentré.

Le mélange réactionnel est porté à 80°C pendant 30 minutes et le précipité orange qui apparait est essoré, lavé à l'eau, séché puis purifié sur colonne Buchi moyenne pression sur silice (éluant : CH₂Cl₂/AcEt : 96/4).Les cristaux jaunes obtenus après évaporation du solvant sont décolorés au charbon activé et recritallisés dans le méthanol.

Cristaux jaunes
**Rdt :** 60%
**F :** >260°C
**Rf :** 0.34 (CH₂Cl₂)
**RMN du** ^{**1**}**H (270 MHz, DMSO d**_{**6**}**) : d (ppm)**
   12.50 (ls, 1H, NH)
   10.83 (ls, 1H, NH)
   8.66 (s, 1H, H-2')
   8.00 (m, 6H, H-4, H-5, H-6, H-7, H-4', H-6')
   7.67 (t, 1H, H-5', JH5'-4' = 7.62Hz)
**RMN du** ^{**13**}**C (270 MHz, DMSO d**_{**6**}**) : d (ppm)**
   186.22 (C=O), 185.61 (C=O), 151.25 (C=O), 148.11 (C-3'), 143.26 (C-2, C-3a, C-7a), 136.22 (C-5, C-6, C-1'), 130.43 (C-2', C-4'), 125.32 (C-5', C-6'), 123.45 (C-4, C-7),
**SM** (I.E) : m/z 338 (M^{+.})
**IR (KBr)**
   υ NH : 3288, 3198 cm⁻¹, C=O : 1727 et 1687 cm^{-1.}

### exemple 3

### 2.3-Dihydro-1.3-dioxo-2-(4-nitrophenylaminocarbonylhydrazono)-1H-indène

A une solution de 8g (0.04 mole) de 2.3-dihydro-2.2-dihydroxy-1.3-dioxo-1H-indène dans 50ml d'éthanol, on ajoute 150ml d'une solution aqueuse fraîchement préparée contenant 7.12g (0.04 mole) de 4-nitrophenylaminocarbonylhydrazine et 0.5ml d'acide chlorhydrique concentré.

Le mélange réactionnel est porté à 80°C pendant 30 minutes et le précipité orange qui apparait est essoré, lavé à l'eau et séché. Les cristaux sont décolorés au charbon activé puis recritallisés dans un grand volume d'acétate d'éthyle.

Cristaux jaunes
**Rdt :** 73%
**F :** >170°C
**Rf :** 0.46 (CH₂Cl₂/AcEt : 90/10)
**RMN du** ^{**1**}**H (270 MHz, DMSO d**_{**6**}**) : d (ppm)**
   12.58 (s, 1H, NH)
   11.00 (s, 1H, NH)
   8.00 (s, 4H, H-4, H-5, H-6, H-7)
   7.84 (d, 2H, H-3', H-5', J H3'-2' = 7.62Hz)
   6.78 (d, 2H, H-2', H-6', J H6'-5' = 7.62Hz)
**RMN du** ^{**13**}**C (270 MHz, DMSO d**_{**6**}**) : d (ppm)**
   186.22 (C=O), 185.61 (C=O), 155.71 (C=O), 150.49 (C-4'), 144.83 (C-1'), 141.04 (C-2, C-3a, C-7a), 136.35 (C-5, C-6), 135.29 (C-2', C-6'), 125.80 (C-3', C-5'), 123.48 (C-4, C-7),
**SM** (I.E) : m/z 338 (M^{+.})
-**IR (KBr)**
   υ NH : 3433, 3232 cm⁻¹, C=O : 1748, 1718 et 1685 cm⁻ 1.

### exemple 4

### 2.3-Dihydro-1.3-dioxo-2-[(2-pyridyl)aminocarbonylhydrazono]-1H-indène

A 4.1g (23 mmoles) de 2.3-dihydro-2,2-dihydroxy-1,3-dioxo-1H-indène en solution dans 50 ml de THF on ajoute à température ambiante 3.5g (23 mmoles) de 2-pyridylaminocarbonylhydrazine en suspension dans 50 ml de THF. Le mélange réactionnel qui prend une coloration jaune est maintenu sous agitation à température ambiante pendant une nuit. Le précipité obtenu est filtré sur verre fritté, lavé à l'éther et purifié sur colonne de silice moyenne pression avec un mélange Heptane/Aceteate d'ethyle, 50/50 pour fournir 4g de 2.3-Dihydro-1.3-dioxo-2-[(2-pyridyl)aminocarbonylhydrazono]-1H-indène sous forme de cristaux jaunes.
- **Rdt :** 59%
- **F :** dégradation
- **Rf :** 0.4 (CH₂Cl₂-MeOH, 98 : 2)
- **RMN du** ^{**1**}**H (270 MHz, DMSO d**_{**6**}**) : d (ppm)**
   14.50 (ls, 1H, NH)
   10.71 (ls, 1H, NH)
   8.44 (d, 1H, H-6', JH-5'-6'=5.5Hz)
   7.97 (m, 5H, H-4, H-5, H-6, H-7, H-3')
   7.83 (dd, 1H, H-4', JH-4'-5'=7.02Hz, JH-3'-4'=7.9Hz)
   7.13 (dd, 1H, H-5', JH-4'-5'=7.02Hz, JH5'-6'=5.19Hz)
- **RMN du** ^{**13**}**C (270 MHz, DMSO d**_{**6**}**) : d (ppm)**
   185.83 (C=O), 185.19 (C=O), 151.65 (C=O, C-2'), 146.99 (C-6'), 139.59 (C-2, C3a, C-7a), 137.21 (C-4'), 136.15 (C-5, C-6), 123,30 (C-4, C-5), 118.72 (C-5'), 112.41 (C-3'),
- **SM (I.E.) :** m/z 294 (M^{+.})
- **IR (KBr)**
   υ NH : 3254 cm⁻¹, C=O : 1732 et 1695cm⁻¹

### exemple 5

### 2.3-Dihydro-1.3-dioxo-2-[(2-pyridyl)aminocarbonylhydrazono]-1H-indène, chlorhydrate

A 260mg (0.88 mmoles) de 2.3-dihydro-1.3-dioxo-2-[(2-pyridyl)aminocarbonylhydrazono]-1H-indène en solution dans 50 ml de méthanol on ajoute à 0°C 25 litres d'acide chlorhydrique en solution dans l'éther (0.9M). Le mélange réactionnel est évaporé sous pression réduite pour fournir 292mg de 2.3-dihydro-1.3-dioxo-2-[(2-pyridyl)aminocarbonylhydrazono]-1H-indène, chlorhydrate sous forme de cristaux jaunes.
- **Rdt** : 100%
- **F** : dégradation
- **Rf** : 0.4 (CH₂Cl₂-MeOH, 98 : 2)
- **RMN du** ^{**1**}**H (270 MHz, DMSO d**_{**6**}**): d (ppm)**
   14.50 (ls, 1H, NH)
   10.72 (ls, 1H, NH)
   8.44 (d, 1H, H-6', JH5'-6'=5.19Hz),
   7.98 (m, 5H, H-4, H-5, H-6, H-7, H-3')
   7.84 (dd, 1H, H-4', JH4'-5'=7.93Hz, JH3'-4'=7.33Hz,)
   7.14 (dd, 1H, H-5', JH4'-5'=7.93Hz, JH5'-6'=5.19Hz),
   3.61 (ls, 1H, NH+)
- **RMN du** ^{**13**}**C (270 MHz, DMSO d**_{**6**}**): d (ppm)**
   185.89 (C=O), 185.19 (C=O), 151.69 (C=O, C-2'), 147.11 (C-4', C-6'), 139.63, (C-2, C3a, C-7a), 136.95 (C-5, C-6), 123,30 (C-4, C-7), 118.74 (C-5'), 112.44 (C-3')
- **IR (KBr)**
   υ NH : 3235cm⁻¹, C=O : 1729 et 1691cm⁻¹

### exemple 6

### 2.3-Dihydro-1.3-dioxo-2-[(2-pyridyl)aminocarbonylhydrazono]-1H-indène, sulfate

A 180mg (0.6 mmole) de 2.3-dihydro-1.3-dioxo-2-[(2-pyridyl)aminocarbonylhydrazono]-1H-indène en solution dans 50 ml de dichlorométhane, on ajoute à 0°C 50.4 litre d'acide sulfurique concentré. Le mélange réactionnel s'eclairci immédiatement et précipite après 5 minutes sous agitation à cette température. Le précipité obtenu est filtré sur verre fritté, lavé à l'éther et séché au pentane pour fournir 784mg de 2.3-dihydro-1.3-dioxo-2-[(2-pyridyl)aminocarbonylhydrazono]-1H-indène, sulfate sous forme de cristaux jaunes.
- **Rdt :** 100%
- **F :** dégradation
- **Rf :** 0.47 (CH₂Cl₂-MeOH, 98 : 2)
- **RMN du** ^{**1**}**H (270 MHz, DMSO d**_{**6**}**) : d (ppm)**
   14.50 (ls, 1H, NH)
   10.80 (ls, 1H, NH)
   8.45 (d, 1H, H-6', JH5'-6'=5.19Hz)
   7.96 (m, 5H, H-4, H-5, H-6, H-7, H-3')
   7.89 (dd, 1H, H-4', JH4'-5'=7.63Hz, JH3'-4'=7.93Hz)
   7.18 (dd, 1H, H-5', JH5'-6'=5.19Hz, JH4'-5'=7.63Hz)
   4.25 (ls, 1H, NH+)
- **RMN du** ^{**13**}**C (270 MHz, DMSO d**_{**6**}**) : d (ppm)**
   185.31 (C=O), 183.05 (C=O), 151.37 (C=O, C-2'), 148.89 (C-6'), 146.51 (C-4'), 140.96 (C-2, C3a, C-7a), 136.25 (C-5, C-6), 123.50 (C-4, C-7), 118 .85 (C-5', C3')
- **IR (KBr)**
   υ NH : 3162cm⁻¹, C=O : 1733 et 1698cm⁻¹

### exemple 7

### 2.3-Dihydro-1.3-dioxo-2-[(3-pyridyl)aminocarbonylhydrazono]-1H-indène

A 5.8g (32.9 mmoles) de 2.3-dihydro-2,2-dihydroxy-1,3-dioxo-1H-indène en solution dans 50 ml d'éthanol on ajoute à température ambiante 5g (32.9 mmoles) de 3-pyridylaminocarbonylhydrazine en suspension dans 50 ml d'éthanol. Le mélange réactionnel qui prend une coloration jaune est maintenu sous agitation à température ambiante pendant 1h30 minutes. Le précipité obtenu est filtré sur verre fritté, lavé à l'éther et rincé au pentane pour fournir 5g de 2.3-dihydro-1.3-dioxo-2-[(3-pyridyl)aminocarbonylhydrazono]-1H-indène sous forme de cristaux jaunes.
- **Rdt :** 51.6%
- **F :** dégradation
- **Rf :** 0.35 (CH₂Cl₂-MeOH, 97 : 3)
- **RMN du** ^{**1**}**H (270 MHz, DMSO d**_{**6**}**) : d (ppm)**
   12.51 (ls, 1H, NH)
   10.54 (ls, 1H, NH)
   8.72 (s, 1H, H-2')
   8.29 (d, 1H, H-6', JH5'-6'=3.51Hz)
   8.00 (ls, 5H, H-4, H-5, H-6, H-7, H-4')
   7.40 (dd, 1H, H-5', JH4'-5'=5Hz, JH5'-6'=3.51Hz)
- **RMN du** ^{**13**}**C (270 MHz, DMSO d**_{**6**}**) : d (ppm)**
   186.59 (C=O), 185.95 (C=O), 151.29 (C=O), 141.2 (C-2, C3a, C-7a), 136.13 (C-5, C-6, C-2', C-3'), 135.33 (C-6'), 131.27 (C-4'), 126.96(C-5'), 123,70 (C-4, C-7)
- **SM (I.E.)** : m/z 294 (M^{+.})
- **IR (KBr)**
   υ NH : 3550 cm⁻¹, C=O : 1724 et 1686 cm⁻¹

### exemple 8

### 2.3-Dihydro-1.3-dioxo-2-[(3-pyridyl)aminocarbonylhydrazono]-1H-indène, chlorhydrate

A 430mg (1.46 mmoles) de 2.3-dihydro-1.3-dioxo-2-[(3-pyridyl)aminocarbonylhydrazono]-1H-indène en solution dans 200 ml de dichlorométhane on ajoute à 0°C 146 litres d'acide chlorhydrique en solution dans l'éther (0.9M). Le mélange réactionnel qui s'eclairci immédiatement est évaporé sous pression réduite pour fournir 482mg de 2.3-dihydro-1.3-dioxo-2-[(3-pyridyl)aminocarbonylhydrazono]-1H-indène, chlorhydrate sous forme de cristaux jaunes.
- **Rdt :** 100%
- **F :** dégradation
- **Rf :** 0.54 (CH₂Cl₂-MeOH, 95 : 5)
- **RMN du** ^{**1**}**H (270 MHz, DMSO d**_{**6**}**) : d (ppm)**
   12.54 (ls, 1H, NH)
   11.11 (ls, 1H, NH)
   9.04 (s, 1H, H-2')
   8.54 (d, 1H, H6', JH5'-6'=4.58Hz)
   8.43 (d, 1H, H4', JH4'-5'=8.24Hz)
   8.00 (s, 4H, H-4, H-5, H-6, H-7)
   7.82 (m, 1H, H-5')
   3.61 (ls, 1H, NH+)
- **RMN du** ^{**13**}**C (270 MHz, DMSO d**_{**6**}**) : d (ppm)**
   186.38 (C=O) , 185.30 (C=O), 151.07 (C=O) , 139.46 (C-2, C3a, C-7a), 137.16 (C-2', C-3'), 136.63 (C-5, C-6), 133.50 (C-4', C-6'), 127.67 (C-5'), 123.50 (C-4, C-7)
- **IR (KBr)**
   υ NH : 3209 et 3450cm⁻¹, C=O : 1736 et 1687cm⁻¹

### exemple 9

### 2.3-Dihydro-1.3-dioxo-2-[(3-pyridyl)aminocarbonylhydrazono]-1H-indène, sulfate

A 600mg (2 mmoles) de 2.3-dihydro-1.3-dioxo-2-[(3-pyridyl)aminocarbonylhydrazono]-1H-indène en solution dans 800 ml d'un mélange 1/1 dichlorométhane/méthanol, on ajoute à 0°C 160 l d'acide sulfurique concentré. Le mélange réactionnel s'eclairci immédiatement et précipite après 5 minutes sous agitation à cette température. Le précipité obtenu est filtré sur verre fritté, lavé à l'éther et rincé au pentane pour fournir 784mg de 2.3-dihydro-1.3-dioxo-2-[(3-pyridyl)aminocarbonylhydrazono]-1H-indène, sulfate sous forme de cristaux jaunes.
- **Rdt :** 100%
- **F :** dégradation
- **Rf :** 0.62 (CH₂Cl₂-MeOH, 95 : 5)
- **RMN du** ^{**1**}**H (270 MHz, DMSO d**_{**6**}**) : d (ppm)**
   12.52 (ls, 1H, NH)
   11.16 (1s, 1H, NH)
   9.17 (s, 1H, H-2')
   8.64 (d, 1H, H-6', JH5'-6'=4.58Hz)
   8.55 (d, 1H, H-4', JH5'-6'=8.24Hz)
   8.00 (s, 5H, H-4, H-5, H-6, H-7, H-5')
   6.00 (1s ,1H, NH+)
- **RMN du** ^{**13**}**C (270 MHz , DMSO d**_{**6**}**) : d (ppm)**
   186.38 (C=O), 185.30 (C=O), 151.07 (C=O),139.46(C-2, C3a, C-7a) , 137.16 (C-2' , C-3'), 136.63 (C-5, C-6) , 133.50 (C-4', C-6'), 127.67 (C-5'), 123.50 (C-4, C-7)
- **IR (KBr)**
   υ NH : 3329 3132 et 3092cm⁻¹, C=O : 1732 et 1674cm⁻¹

### exemple 10

### 2.3-Dihydro-1.3-dioxo-4-fluoro-2-(phenylaminocarbonylhydrazono)-1H-indène

A une solution de 0.900g (4.6 mmoles) de 2.3-dihydro-2,2-dihydroxy-1,3-dioxo-4-fluoro-1H-indène dans 5ml d'éthanol,on ajoute à chaud 3ml d'une solution aqueuse fraîchement préparée contenant 0.86g (4.6mmoles) de phenylaminocarbonylhydrazine et 0.5ml d'acide chlorhydrique concentré.

Le mélange réactionnel est porté au reflux pendant 2h30minutes et le précipité jaune qui apparait est essoré à température ambiante puis séché. Les cristaux jaunes obtenus sont décolorés au charbon activé puis recristallisés dans un grand volume de dichlorométhane.

Cristaux jaunes.
- **Rdt :** 85%
- **F :** 204°C
- **Rf :** 0.5 (CH₂Cl₂/AcEt : 88/12)
   **SM** (I.E) : m/z 311 (M^{+.})
   **RMN du** ^{**1**}**H (270 MHz, DMSO d**_{**6**}**) : d (ppm)**
   12.54 (ls, 1H, NH)
   10.40 (ls, 1H, NH)
   7.97 (m, 1H, H-6)
   7.82 (m, 2H, H-5, H-7)
   7.56 (d, 2H, H-2', H-6', J H2'-3' = J H5'-6'=7.63Hz)
   7.36 (m, 2H, H-3', H-5')
   7.09 (m, 1H, H-4')
   **RMN du** ^{**13**}**C (270 MHz, DMSO d**_{**6**}**) : d (ppm)**
   185.62 (C=O),185.14 (C=O) , 165.09 (C-4), , 151.90 (C=O), 150.30 (C-1'), 138.26 (C-2, C-7a), 134.70 (C-3a), 129.02 (C-3', C-5'), 123.51 (C-6, C-7, C-4'), 119.14 (C-5, C-2', C-6')
   **IR (KBr)**
   υ NH : 3295, 3240 cm⁻¹, C=O : 1740 et 1690 cm^{-1.}

### exemple 11

### 2.3-Dihydro-1.3-dioxo-4-fluoro-2-(4-fluorophenylaminocarbonylhydrazono)-1H-indène

A une solution de 0.600g (3.0 mmoles) de 2,3-dihydro-2,2-dihydroxy-1,3-dioxo-4-fluoro-1H-indène dans 5ml d'éthanol,on ajoute à chaud 3ml d'une solution aqueuse fraîchement préparée contenant 0.616g (3.0mmoles) de 4-fluorophenylaminocarbonylhydrazine. Le mélange réactionnel est porté au reflux pendant 30 minutes et le précipité jaune qui apparait est essoré à température ambiante, séché, décoloré au charbon activé puis recristallisé dans un grand volume de dichlorométhane.

Cristaux jaunes.
**Rdt :** 68%
**F :** 215°C
**Rf :** 0.5 (CH₂Cl₂/AcEt : 88/12)
**RMN du** ^{**1**}**H (270 MHz, DMSO d**_{**6**}**) : d (ppm)**
   12.49 (ls, 1H, NH)
   10.44 (ls, 1H, NH)
   7.99 (m, 1H, H-6)
   7.81 (m ,2H, H-5, H-7)
   7.56 (m ,2H, H-3', H-5')
   7.22 (m, 2H, H-2', H-6')
**RMN du** ^{**13**}**C (270 MHz, DMSO d**_{**6**}**) : d (ppm)**
   186.19 (C=O), 184.69 (C=O), 159.05 (C-4, C-4'), 151.83 (C=O), 148.25 (C-1'), 141.97 (C-7a), , 136.65 (C-2, C-3a), 134.56 (C-7), 123.54 (C-6), 121.05 (C-2', C-6'), 119.80 (C-5), 115.56 (C-3', C-5')
**SM** (I.E) : m/z 311 (M^{+.})
**IR (KBr)**
   υ NH : 3290, 3205 cm⁻¹, C=O : 1700 et 1620 cm^{-1.}

### exemple 12

### 2.3-Dihydro-1.3-dioxo-4-fluoro-2-[(3-pyridyl)aminocarbonylhydrazono]-1H-indène

A une solution de 0.500g (2.5 mmoles) de 2.3-dihydro-2,2-dihydroxy-1,3-dioxo-4-fluoro-1H-indène dans 10ml d'éthanol, on ajoute une solution aqueuse fraîchement préparée contenant 0.380g (2.5mmoles) de 3-pyridylaminocarbonylhydrazine et 5ml d'eau.

Le mélange réactionnel est agité à température ambiante pendant deux heures et le précipité rouille qui apparait est essoré, séché, décoloré puis purifié sur colonne de silice (CH₂Cl₂/MeOH : 99/1).

Cristaux jaunes
**Rdt :** 40%
**F :** déc. 215°C
**Rf :** 0.6 (CH₂Cl₂/MeOH : 96/4)
**RMN du** ^{**1**}**H (270 MHz, DMSO d**_{**6**}**) : d (ppm)**
   12.52 (ls, 1H, NH)
   10.57 (ls, 1H, NH)
   8.73 (s, 1H, H-2')
   8.31 (d, 1H, H-6', J H6'-5' = 4.2 Hz)
   8.05 (m, 2H, H-7, H-4')
   7.80 (m, 2H, H-5, H-6)
   7.41 (m ,1H, H-5')
**RMN du** ^{**13**}**C (270 MHz, DMSO d**_{**6**}**) : d (ppm)**
   185.62 (C=O), 185.14 (C=O), 165.09 (C-4), 151.05 (C=O), 144.45 (C-3'), 140.80 (C-2, C-7a), 138.49 (C-2', C-6'), 135.17 (C-3a), 126.39 (C-5'), 123.70 (C-6, C-7), 119.92 (C-5), 119.48 (C-4')
**SM** (I.E) : m/z 312 (M^{+.})
**IR (KBr)**
   υ NH : 3290 et 3184 cm⁻¹, C=O : 1740, 1730 et 1620 cm^{-1.}

### exemple 13

### 2.3-Dihydro-1.3-dioxo-4-fluoro-2-[(3-pyridyl)aminocarbonylhydrazono]-1H-indène, chlorhydrate.

A une solution de 0.200g (0.6 mmole) de 2.3-dihydro-1.3-dioxo-4-fluoro-2-(3-pyridylaminocarbonylhydrazono)-1H-indène dans 60ml de méthanol, on ajoute 0.6ml d'une solution méthanolique d'acide chlorhydrique 1M.

Le mélange réactionnel est agité à température ambiante pendant 15minutes et le précipité beige obtenu est essoré.

Cristaux beiges
**Rdt :** 96%
**F :** >215°C
**Rf :** 0.6 (CH₂CL₂/MeOH : 95/5)
**RMN du** ^{**1**}**H (270 MHz, DMSO d**_{**6**}**) : d (ppm)**
   12.52 (ls, 1H, NH)
   10.85 (1s, 1H, NH)
   8.95 (s, 1H, H-2')
   8.48 (d, 1H, H-6', J H6'-5' = 4.58 Hz)
   8.35 (d, 1H, H-4', JH4'-5'=8.24Hz)
   8.04 (m, 1H, H-7)
   7.82 (m, 2H, H-5, H-6)
   7.75 (m ,1H, H-5')
   3.41 (1s, 1H, NH+)
**RMN du** ^{**13**}**C (270 MHz, DMSO d**_{**6**}**): d (ppm)**
   186.19 (C=O), 184.69 (C=O), 159.05 (C-4, C-4'), 151.83 (C=O), 148.25 (C-1'), 141.97 (C-7a), 136.65 (C-2, C-3a), 134.56 (C-7), 123.54 (C-6), 121.05 (C-2' , C-6'), 119.80 (C-5), 115.56 (C-3', C-5')
**IR (KBr)**
   υ NH : 2694, 2635 cm⁻¹, C=O : 1737 et 1687 cm⁻¹.

### exemple 14

### 2.3-Dihydro-1.3-dioxo-5-fluoro-2-(phenylaminocarbonylhydrazono)-1H-indène

A une solution de 1g (5.0 mmoles) de 2.3-dihydro-2,2-dihydroxy-1,3-dioxo-5-fluoro-1H-indène dans 10ml d'éthanol, on ajoute une solution fraîchement préparée contenant 0.94g (5.0 mmoles) de phenylaminocarbonylhydrazine 5ml d'eau et 0.3ml d'acide chlorhydrique concentré.

Le mélange réactionnel est porté une heure au reflux et le précipité orange qui apparait est essoré à température ambiante, séché et purifié sur colonne de silice Eluant (CH₂Cl₂/Hept. : 99/1)

Cristaux jaunes.
**Rdt** : 80%
**F** : >260°C
**Rf** : 0.5 (CH₂Cl₂/MeOH : 98/2).
**RMN du** ^{**1**}**H (270 MHz, DMSO d**_{**6**}**): d (ppm)**
   12.50 (ls, 1H, NH)
   10.40 (s, 1H, NH)
   8.00 (m, 1H, H-7)
   7.83 (m, 2H, H-4, H-6)
   7.58 (d, 2H, H-2', H-6', J H2'-6' = 7.62Hz)
   7.36 (m, 2H, H-3', H-5')
   7.09 (m, 1H, H-4')
**RMN du** ^{**13**}**C (270 MHz, DMSO d**_{**6**}**): d (ppm)**
   186.53 (C=O), 185.67 (C=O), 150.33 (C=O), 144.50 (C-138.26 (C-5), 137.57 (C-2, C-3a, C-7a), 129.04 (C-3', C-5'), 125.55 (C-7), 123.55 (C-4'), 119.21 (C-2', C-6'), 110.50 (C-4, C-6)
**SM** (I.E) : m/z 311 (M^{+.})
**-IR (KBr)**
   υ NH : 3390, 3330 cm⁻¹, C=O : 1745, 1725 et 1690 cm⁻ 1.

### exemple 15

### 2.3-Dihydro-1.3-dioxo-5-fluoro-2-[(4-fluorophenyl)aminocarbonylhydrazono)]-1H-indène

A une solution de 1g (5.0 mmoles) de 2.3-dihydro-2,2-dihydroxy-1,3-dioxo-5-fluoro-1H-indène dans 10ml d'éthanol,on ajoute une solution fraîchement préparée contenant 0.94g (5.0mmoles) de (4-fluorophenyl)aminocarbonylhydrazine, 5ml d'eau et 0.3ml d'acide chlorhydrique concentré.

Le mélange réactionnel est porté une heure au reflux et le précipité orange qui apparait est essoré à température ambiante, séché et purifié sur colonne de silice Eluant (CH₂Cl₂/Hept. : 98/2)

Cristaux jaunes
**Rdt :** 60%
**F :** >260°C
**Rf :** 0.5 (CH₂Cl₂/MeOH : 98/2)
**RMN du** ^{**1**}**H (270 MHz, DMSO d**_{**6**}**) : d (ppm)**
   12.45 (ls, 1H, NH)
   10.41 (s, 1H, NH)
   8.07 (m ,1H, H-7)
   7.80 (m ,2H, H-4, H-6)
   7.60 (m, 2H, H-3', H-5')
   7.58 (t, 2H, H-2', H-6' , J H-5',6' = 9.00 Hz)
**RMN du** ^{**13**}**C (270 MHz, DMSO d**_{**6**}**) : d (ppm)**
   186.53(C=O), 185.67 (C=O), 162.45 (C-4'), 150.86 (C=O), 145.50 (C-1'), 134.89 (C-2, C-5, C-3a, C-7a), 128.50 (C-7), 121.54 (C-2', C-6'), 116.12 (C-3', C-5'), 110.35 (C-4, C-6)
**SM** (I.E) : m/z 329 (M^{+.})
**-IR (KBr)**
   υ NH : 3240 cm⁻¹, C=O : 1740 et 1700 cm^{-1.}

### exemple 16

### 2.3-Dihydro-1.3-dioxo-5-fluoro-2-[(3-pyridyl)aminocarbonylhydrazono]-1H-indène

A 1g (5.6 mmoles) de 2.3-dihydro-2.2-dihydroxy-1.3-dioxo-5-fluoro-1H-indène en solution dans 50 ml d'éthanol on ajoute à température ambiante 0.85g (5.6 mmoles) de 3-pyridylaminocarbonylhydrazine en suspension dans 50 ml d'éthanol. Le mélange réactionnel qui prend une coloration jaune est maintenu sous agitation à température ambiante pendant 1h30minutes. Le précipité obtenu est filtré sur verre fritté, lavé à l'éther et purifié sur colonne de silice moyenne pression avec un mélange Heptane/Acetate d'éthyle 50/50 pour fournir 1.2g de 2.3-Dihydro-1.3-dioxo-5-fluoro-2-[(3-pyridyl)aminocarbonylhydrazono]-1H-indène sous forme de cristaux jaunes.
- **Rdt :** 69%
- **F :** dégradation
- **Rf :** 0.59 (CH₂Cl₂-MeOH, 95 : 5)
- **RMN du** ^{**1**}**H (270 MHz, DMSO d**_{**6**}**) : d (ppm)**
   12.47 (ls, 1H, NH)
   10.54 (ls, 1H, NH)
   8.72 (s, 1H, H-2')
   8.30 (d, 1H, H-6', JH5'-6'=3.51Hz)
   8.04 (m, 2H, H-7, H-4')
   7.80 (m, 2H, H-4, H-6)
   7.39 (m, 1H, H-5')
- **RMN du** ^{**13**}**C (270 MHz, DMSO d**_{**6**}**) : d (ppm)**
   186.59 (C=O), 185.95 (C=O), 151.29 (C=O), 144.32 (C-5), 140.96 (C-2, C-3', C-3a, C-7a), 135.29 (C-2', C-6'), 126.28(C-4', C-5'), 123.80 (C-7), 110.04 (C-4, C-6)
- **SM (I.E.)** : m/z 313 (MH+)
- **IR (KBr)**
   υ NH : 3554, 3250 cm⁻¹, C=O : 1730 et 1691 cm⁻¹

### exemple 17

### 2.3-Dihydro-1.3-dioxo-5-fluoro-2-[(3-pyridyl)aminocarbonylhydrazono]-1H-indène, sulfate

A 250mg (0.8 mmole) de 2.3-Dihydro-1.3-dioxo-5-fluoro-2-[(3-pyridyl)aminocarbonylhydrazono]-1H-indène en solution dans 50 ml d'un mélange 1/1 dichlorométhane/méthanol on ajoute à 0°C 66 l d'acide sulfurique concentré. Le mélange réactionnel s'eclairci immédiatement et précipite après 5 minutes sous agitation à cette température. Le précipité obtenu est filtré sur verre fritté, lavé à l'éther et séché au pentane pour fournir 328mg de 2.3-Dihydro-1.3-dioxo-5-fluoro-2-[(3-pyridyl)aminocarbonylhydrazono]-1H-indène, sulfate sous forme de cristaux jaunes.
- **Rdt :** 100%
- **F :** 250°C
- **Rf :** 0.65 (CH₂Cl₂-MeOH, 95 : 5)
- **RMN du** ^{**1**}**H (270 MHz, DMSO d**_{**6**}**) : d (ppm)**
   12.49 (ls, 1H, NH)
   11.04 (ls, 1H, NH)
   9.08 (s, 1H, H-2')
   8.56 (d, 1H, H-6', JH5'-6'=4.4Hz)
   8.46 (d, 1H, H-4', JH4'-5'=8.26Hz)
   8.43 (m, 1H, H-7)
   7.86 (m, 3H, H-4, H-6, H-5')
   4.48 (ls, 1H, NH+)
- **RMN du** ^{**13**}**C (270 MHz, DMSO d**_{**6**}**) : d (ppm)**
   173.10 (C=O), 165.10 (C=O), 160.05 (C-5), 151.02 (C=O), 144.82 (C-4'), 140.05 (C-6'), 138.25 (C-2, C-7a), 137.22 (C-2', C 3'), 133.03 (C-2), 127.23 (C-5'), 123.78 (C-7), 111.12 (C-4, C-6)
- **IR (KBr)**
   υ NH : 3351 et 3207cm⁻¹, C=O : 1732 et 1690cm⁻¹

### exemple 18

### 2.3-Dihydro-1.3-dioxo-4-nitro-2-(4-phenylaminocarbonylhydrazono)-1H-indène

A 2g (9.76 mmoles) de 2.3-Dihydro-2.2-dihydroxy-1.3-dioxo-4-nitro-1H-indène en solution dans 100 ml d'éthanol on ajoute à température ambiante 1.47g (9.76 mmoles) de 4-Phenylaminocarbonylhydrazine en suspension dans 50 ml d'éthanol et quelques gouttes acide chlorhydrique. Le mélange réactionnel qui prend une coloration orangée est maintenu sous agitation à température ambiante pendant 2h. Le précipité obtenu est filtré sur verre fritté et purifié sur colonne de silice moyenne pression avec un mélange CH₂Cl₂/MeOH, 99.5/0.5 pour fournir 2g de 2.3-Dihydro-1.3-dioxo-4-nitro-2-(4-phenylaminocarbonylhydrazono)-1H-indène sous forme de cristaux jaunes.
- **Rdt** : 61%
- **F** : 236°C
- **Rf** : 0.54 (CH₂Cl₂-MeOH, 99 : 1)
- **RMN du** ^{**1**}**H (270 MHz, DMSO d**_{**6**}**) : d (ppm)**
   12.61 (ls, 1H, NH)
   10.45 (ls, 1H, NH)
   8.33 (d, 1H, H-5, JH5-6=7.32Hz)
   8.23 (d, 1H, H-7, JH6-7=7.48Hz)
   8.16 (m, 1H, H-6)
   7.57 (d, 2H, H-2', H-6', JH2'-3'=JH5'-6'=8.24Hz)
   7.36 (dd, 2H, H-3', H-5', JH2'-3' et JH5'-6'=8.24Hz, JH3'-4' et JH4'-5'=7.33Hz)
   7.09 (m, 1H, H-4')
- **RMN du** ^{**13**}**C (270 MHz, DMSO d**_{**6**}**) : d (ppm)**
   185.75 (C=O), 182.50 (C=O), 150.05 (C-4), 150.27 (C=O, C-2', C-6'), 145.86 (C-4'), 137.33 (C-2, C-7a), 129.40 (C-7), 126.46 (C-6, C-3a), 123.52 (C-5), 113.81 (C-3', C-5')
- **SM (I.E.)** : m/z 338 (M+.)
- **IR (KBr)**
   υ NH : 3416 et 3324 cm⁻¹, C=O : 1742 1725et 1696 cm⁻¹, NO2 : 1535 cm⁻¹

### exemple 19

### 2.3-Dihydro-1.3-dioxo-4-nitro-2-[4-(4-fluorophenyl)aminocarbonylhydrazono]-1H-indène

A 0.8g (3.90 mmoles) de 2.3-Dihydro-2.2-dihydroxy-1.3-dioxo-4-nitro-1H-indène en solution dans 50 ml d'éthanol on ajoute à température ambiante 0.6g (3.90 mmoles) de 4-(4-Fluorophenyl) aminocarbonylhydrazine en suspension dans 50 ml d'éthanol et quelques gouttes d' acide chlorhydrique. Le mélange réactionnel qui prend une coloration orangée est maintenu sous agitation à température ambiante pendant 15mn. Le précipité obtenu est filtré sur verre fritté et purifié sur colonne de silice moyenne pression avec du CH₂Cl₂ pour fournir 1g de 2.3-Dihydro-1.3-dioxo-4-nitro-2-[4-(4-fluorophenyl) aminocarbonylhydrazono]-1H-indène sous forme de cristaux jaunes.
- **Rdt** : 72%
- **F** : >260°C
- **Rf** : 0.54 (CH₂Cl₂-MeOH, 98 : 2)
- **RMN du** ^{**1**}**H (270 MHz, DMSO d**_{**6**}**): d (ppm)**
   12.56 (ls, 1H, NH)
   10.47 (ls, 1H, NH)
   8.33 (d, 1H, H-5, JH5-6=7.94Hz)
   8.21 (d, 1H, H-7, JH6-7=7.78Hz)
   8.16 (m, 1H, H-6)
   7.58 (m, 2H, H-3', H-5')
   7.20 (m, 2H, H-2', H-6')
- **RMN du** ^{**13**}**C (270 MHz, DMSO d**_{**6**}**): d (ppm)**
   185.98 (C=O), 182.50 (C=O), 159.05 (C-4'), 150.32 (C=O, C-4), 148.25 (C-1'), 137.33 (C-2, C-7a), 134.95 (C-2', C-6'), 129.35 (C-7), 126.76 (C-6, C-3a), 123.52 (C-5), 114.90 (C-3', C-5')
- **SM (I.E.)** : m/z 356 (M+.)
- **IR (KBr)**
   υ NH : 3351 et 3212 cm⁻¹, C=O : 1736 1725et 1699 cm⁻¹, NO2 : 1509 cm⁻¹

### exemple 20

### 2.3-Dihydro-1.3-dioxo-4-nitro-2-[(3- pyridyl)aminocarbonylhydrazono]-1H-indène

A 3g (14.6 mmoles) de 2.3-Dihydro-2.2-dihydroxy-1.3-dioxo-4-nitro-1H-indène en solution dans 50 ml d'éthanol on ajoute à température ambiante 2.2g (14.6 mmoles) de 3-pyridylaminocarbonylhydrazine en solution dans 50 ml d'éthanol. Le mélange réactionnel qui prend une coloration jaune est maintenu sous agitation à température ambiante pendant 15minutes. Le précipité obtenu est filtré sur verre fritté, purifié sur colonne de silice moyenne pression avec un mélange CH₂Cl₂/MeOH, 98/21 pour fournir 3g de 2.3-Dihydro-1.3-dioxo-4-nitro-2-[(3-pyridyl)aminocarbonylhydrazono]-1H-indène sous forme de cristaux jaunes.
- **Rdt :** 60.6%
- **F** : dégradation
- **Rf** : 0.46 (CH₂Cl₂-MeOH, 96 : 4)
- **RMN du** ^{**1**}**H (270 MHz, DMSO d**_{**6**}**) : d (ppm)**
   12.75 (ls, 1H, NH)
   10.85 (ls, 1H, NH)
   8.84 (ls, 1H, H-2')
   8.29 (m, 2H, H-5, H-6')
   8.11 (m, 3H, H-6, H-7, H-4')
   7.39 (m, 1H, H-5')
- **RMN du** ^{**13**}**C (270 MHz, DMSO d**_{**6**}**): d (ppm)**
   181.90 (C=O), 179.55 (C=O), 151.84 (C=O), 145.40 (C-4), 142.50 (C-3'), 139.35 (C-2'), 138.95 (C-7a), 138.26 (C-2), 137.50 (C-6'), 129.90 (C-7), 127.27 (C-6, C-3a), 125.35 (C-5, C-4', C-5')
- **SM (I.E.)** : m/z 339
- **IR (KBr)**
   υ NH : 3550 cm⁻¹, C=O : 1698 cm⁻¹

### exemple 21

### 2.3-Dihydro-1.3-dioxo-4-nitro-2-[(3-pyridyl)aminocarbonylhydrazono]-1H-indène, sulfate

A 108mg (0.3 mmole) de 2.3-Dihydro-1.3-dioxo-4-nitro-2-[(3-pyridyl)aminocarbonylhydrazono]-1H-indène en solution dans 50 ml d'éthanol on ajoute à température ambiante 26 1 (0.3 mmole) d'acide sulfurique. Le milieu réactionnel qui prend une coloration jaune est maintenu sous agitation à 0°C pendant 10minutes. Le précipité obtenu est filtré sur verre fritté, lavé à l'éther et rincé au pentane pour fournir 139mg de 2.3-Dihydro-1.3-dioxo-4-nitro-2-[(3-pyridyl)aminocarbonylhydrazono]-1H-indène, sulfate sous forme de cristaux jaunes
- **Rdt** : 100%
- **F** : dégradation
- **Rf** : 0.46 (CH₂Cl₂-MeOH, 96 : 4)
- **RMN du** ^{**1**}**H (270 MHz, DMSO d**_{**6**}**) : d (ppm)**
   12.65 (ls, 1H, NH)
   10.89 (ls, 1H, NH)
   8.93 (ls, 1H, H-2')
   8.35 (d, 1H, H-6', JH5'-6'=4.58Hz)
   8.27 (m, 1H, H-4')
   8.24 (m, 1H, H-5)
   8.16 (m, 2H, H-6, H-7)
   7.70 (m, 1H, H-5)
   5.75 (ls, 1H, NH+)
- **RMN du** ^{**13**}**C (270 MHz, DMSO d**_{**6**}**) : d (ppm)**
   182.55 (C=O), 181.65 (C=O), 150.59 (C=O), 145.95(C-4), 145.85(C-4'), 142.25 (C-2), 140.05 (C-6'), 139.77 (C-2, C-7a), 137.57 (C-7), 135.55 (C-3'), 130.15 (C-3a), 129.45 (C-6), 126.85 (C-5, C-5')
- **IR (KBr)**
   υ NH : 3550 et 2917cm⁻¹, C=O : 1750, 1696 cm⁻¹, NO2: 1541 cm⁻¹

### exemple 22

### 2.3-Dihydro-1.3-dioxo-5-nitro-2-(phenylaminocarbonylhydrazono)-1H-indène

A 1g (4.9 mmoles) de 2.3 dihydro-2,2-dihydroxy-1,3-dioxo-5-nitro-1H-indène en solution dans 50 ml d'éthanol on ajoute à température ambiante 2.2g (14.6 mmoles) de 4-phenylaminocarbonylhydrazine en suspension dans 50 ml d'éthanol et quelques gouttes d'acide chlorhyrique. Le mélange réactionnel qui prend alors une coloration orangée est maintenu sous agitation à température ambiante pendant 1h30 minutes. Le précipité obtenu est filtré sur verre fritté et purifié sur colonne de silice moyenne pression avec un mélange CH₂Cl₂/MeOH, 98/2 pour fournir 1g de 2.3-dihydro-1.3-dioxo-5-nitro-2-(phenylaminocarbonylhydrazono)-1H-indène sous forme de cristaux jaunes.
- **Rdt** : 60%
- **F** : >260°C
- **Rf** : 0.46 (CH₂Cl₂-MeOH, 96 : 4)
- **RMN du 1**^{**H**} **(270 MHz, DMSO d**_{**6**}**): d (ppm)**
   12.59 (ls, 1H, NH)
   10.50 (ls, 1H, NH)
   8.67 (d, 1H, H-6, JH6-7=8.55Hz)
   8.56 (s, 1H, H-4)
   8.20 (d, 1H, H-7, JH6-7=8.55Hz)
   7.57 (d, 2H, H-2', H-6', JH2'-3' et JH5'-6'=7.94Hz
   7.37 (dd, 2H, H-3', H-5', JH2'-3' =JH5'-6'=7.94Hz, JH3'-4' =JH4'-5'=6.41Hz)
   7.11 (m, 1H, H-4')
- **RMN du** ^{**13**}**C (270 MHz, DMSO d**_{**6**}**): d (ppm)**
   185.75 (C=O), 182.50 (C=O), 151.73 (C=O), 150.08 (C-5), 148.50 (C-1') , 138.18 (C-2), 135.75 (C-3a, C-7a), 131.60 (C-7), 129.08 (C-3', C-5'), 125.40 (C-4'), 123.63 (C-4, C-6), 119.10 (C-2', C-6')
- **SM (I.E.)** : m/z 338 (M+.)
- **IR (KBr)**
   υ NH : 3422 et 3217 cm⁻¹, C=O : 1731 et 1693 cm^{-1,} NO2 : 1529 cm⁻¹

### exemple 23

### 2.3-Dihydro-1.3-dioxo-2-(t-butyloxycarbonylhydrazono)-1H-indène

A une solution de 5.34g (0.03 mole) de 2.3-dihydro-2.2-dihydroxy-1.3-dioxo-1H-indène dans 50ml d'éthanol, on ajoute 3.96g (0.03 mole) de terbutylcarbazate en solution dans l'éthanol.

Le mélange réactionnel est porté à 80°C pendant trois heures. Le précipité jaune qui apparait après refroidissement à température ambiante est essoré, séché, décoloré au charbon activé puis recritallisé dans un mélange éther éthylique/méthanol(40/4ml).

Cristaux jaunes
**Rdt** : 71%
**F** : 170°C
**Rf** : 0.5 (CH₂Cl₂/MeOH : 98/2)
**RMN du** ^{**1**}**H (270 MHz, DMSO d**_{**6**}**) : d (ppm)**
   12.06 (s, 1H, NH)
   8.00 (s, 4H, H-4, H-5, H-6, H-7)
   1.53 (ls, 9H, CH₃)
**SM (I.E)** : m/z 274 (M^{+.})
-**IR (KBr)**
   v NH : 3230 cm⁻¹, C=O : 1770,1740 et 1700 cm^{-1.}

### exemple 24

### 2.3-Dihydro-1.3-dimethylimino-2-(t-butyloxycarbonylhydrazono)-1H-indène

A une suspension de 0.400g (1.4 mmoles) de 2.3-dihydro-1.3-dioxo-2-(t-butyloxycarbonylhydrazono)-1H-indène dans 5ml d'éthanol,on ajoute 1ml d'une solution aqueuse de méthylamine à 40%.

Le mélange réactionnel est agité à température ambiante pendant une nuit et le précipité jaune-clair qui apparait est filtré, lavé à l'eau et séché. Les cristaux blancs obtenus sont décolorés au charbon activé puis recritallisés dans le dichlorométhane.

Cristaux jaunes
**Rdt :** 57%
**F :** 174°C
**Rf :** 0.5 (CH₂Cl₂/MeOH : 98/2)
**RMN du** ^{**1**}**H (270 MHz, DMSO d**_{**6**}**) : d (ppm)**
   12.00 (ls, 1H, NH)
   8.00 (s ,4H, H-4, H-5, H-6, H-7)
   1.53 (ls, 9H, CH₃)
**SM** (I.E) : m/z 300 (M^{+.})
**-IR (KBr)**
   υ CH₃, = 2990 cm^{-1.,} C=O : 1740 et 1660 cm^{-1.}

### exemple 25

### 2.3-Dihydro-1.3-dimethylimino-2-(methylaminocarbonylhydrazono)-1H-indène

Une suspension de 5.80g (21mmoles) de 2.3-dihydro-1.3-dioxo-2-(t-butyloxycarbonylhydrazono)-1H-indène dans 300 ml d'une solution aqueuse de méthylamine à 40% est agitée à température ambiante pendant cinq heures et le précipité gris-vert qui apparait est filtré, lavé à l'éther éthylique et séché. Les cristaux obtenus sont décolorés au charbon activé puis recritallisés dans le dichlorométhane.

Cristaux gris-vert
**Rdt :** 55%
**F :** 220°C
**Rf :** 0.6 (CH₂Cl₂/MeOH : 98/2)
**RMN du** ^{**1**}**H (270 MHz, DMSO d**_{**6**}**) : d (ppm)**
   11.44 (s, 2H, NH)
   8.23 (m, 2H, H-4, H-7)
   8.00 (m, 2H, H-5, H-6)
   4.20 (s, 3H, CH₃)
   3.76 (s, 3H, CH₃)
   2.50 (d, 3H, CH₃)
**SM (I.E)** : m/z 257(M^{+.})
-**IR (KBr)**
   υ NH : 3220; CH₃, = 2990 cm^{-1,} C=O : 1675 cm^{-1.}

### exemple 26

### 2.3-Dihydro-1.3-dimethylimino-2-(phenylaminocarbonylhydrazono)-1H-indène

1g (3.4 mmoles) de 2,3-dihydro-1,3-dioxo-2-(phenylaminocarbonylhydrazono)-1H-indène est mis en suspension dans 75ml d'une solution aqueuse de méthylamine à 40%. Le mélange réactionnel est agité à température ambiante pendant 24 heures et le précipité rouge orangé qui apparait est filtré, lavé à l'eau et séché. Les cristaux roses obtenus sont décolorés au charbon activé puis recritallisés dans l'éthanol.

Cristaux jaunes
**Rf :** 0.2 (CH₂Cl₂/MeOH : 96/4)
**Rdt** : 61%
**F** : 180°C
**RMN du** ^{**1**}**H (270 MHz, DMSO d**_{**6**}**) : d (ppm)**
   12.50 (s, 1H, NH)
   9.71 (s, 1H, NH)
   8.47 (d, 1H, H-7, J H7-6 = 8Hz)
   8.27 (d, 1H, H-4', J H4-5 = 8Hz)
   8.02 (t, 2H, H-5, H-6, J H5-6 = 8Hz)
   7.93 (d, 2H, H-2', H-6' ,J H2'-3' = 8Hz)
   7.68 (t, 2H, H-3', H-5', J H5'-6' = 8Hz)
   7.41 (t ,1H, H-4', J H4'-6' = 8Hz)
   4.25 (ls, 3H, CH₃)
   4.19 (ls, 3H, CH₃)
**SM (I.E)** : m/z 319 (M^{+.})
-**IR (KBr)**
   υ C=0 : 1660 ; C=N : 1630 et 1600cm⁻¹.

### exemple 27

### 2,3-dihydro-1,3-diméthylimino-2-hydrazono-1H indène

A une mole de 2,3-dihydro-1,3-diméthylimino-2-(t-butyloxycarbonylhydrazono)-1H-indène en solution dans 50 ml de dichlorométhane, on ajoute 2 moles de chlorure de triméthylsilyle, on laisse agiter à température ambiante pendant 24 heures. Le précipité qui apparaît est filtré. On recueille des cristaux qui sont lavés plusieurs fois à l'heptane et qui sont décolorés au charbon activé. Après chromatographie sur colonne de silice, le rendement obtenu est de 80%.

L'étude des composés de la présente invention et de leurs sels éventuels a montré qu'ils possèdent diverses propriétés pharmacologiques. Ainsi, ils sont veinotoniques et n'affectent pas dans la plupart des cas le système artériel ; par ailleurs, ils augmentent la résistance capillaire, diminuent l'hyperperméabilité vasculaire induite par certains agents inflammatoires et présentent des propriétés antilipoperoxydantes, antiradicalaires et antiinflammatoires, ainsi qu'une activité protectrice dans le choc septique.

Ces propriétés sont démontrées chez les mammifères tels que les rats, cobayes et lapins, dans des conditions in vitro (vaisseaux ou réseaux vasculaires isolés) et in vivo.

Pour l'étude in vitro, les composés sont solubilisés en solution aqueuse pure ou contenant du DMSO ou de l'alcool.

Pour l'étude in vivo, ils sont administrés par voie intra-veineuse sous forme de solution aqueuse pure, par voie intrapéritonéale sous forme de solution aqueuse contenant ou non du DMSO ou par voie orale en solution ou en suspension dans la carboxyméthylcellulose ou dans une solution aqueuse composée contenant du Tween^{(R)} et dans certains cas du DMSO (diméthylsulfoxyde).

### Modèles d'études pharmacologiques

L'effet contractile est mesuré in vitro :
- par la force de contraction développée par des anneaux vasculaires quiescents ou stimulés (soit électriquement soit par des agents physiologiques) et maintenus dans des conditions isométriques,
- par la pression développée par des réseaux vasculaires perfusés à débit constant.

In vivo, les pressions artérielles et veineuses sont mesurées dans des conditions normales et après arrêt cardiaque. Lors de l'arrêt cardiaque, le tonus veineux est calculé à partir des pressions veineuses et artérielles mesurées à l'équilibre et corrigées en fonction des différences relatives de compliance entre ces deux réseaux (Samar et Coleman, Am. J. Physiol., 1978, 234 : H94-100 ; Yamamoto et col., Am. J; Physiol., 1980, 238 : H823-828).

L'augmentation de la résistance capillaire est appréciée par la modification de l'index pétéchial (pression négative induisant l'extravasation d'érythrocytes), mesuré par une méthode dérivée de l'angiosterromètre de Parrot.

La perméabilité vasculaire est étudiée in vivo et in vitro par la mesure de l'extravasation d'albumine ou de colorant liant l'albumine (Bleu Evans). In vivo, l'hyperperméabilité est induite par l'injection d'une solution d'histamine, de bradykinine ou de zymosan. Les modèles in vitro permettent de réaliser des hyperpressions (sur un territoire vasculaire isolé) et/ou des réactions vasculaires inflammatoires.

L'activité antiinflammatoire est démontrée par la mesure de l'inhibition de l'oedème et de la migration leucocytaire après induction d'une pleurésie chez le rat par injection de carragénine dans la cavité pleurale (Almeida et col., J. Pharmacol. Exp. Therap., 1980, 214 : 74).

L'effet "piégeur de radicaux libres" global est étudié in vitro par un modèle utilisant le 1,1-diphényl-2-picrylhydrazyl (DPPH) comme radical libre stable, méthode dérivée de celle décrite par Lamaison et col., Plantes Médic. et Phytothérapie, XXII, 1988, 231-234.

L'activité antioxydante est étudiée in vitro par un modèle de peroxydation lipidique basée sur la peroxydation d'une émulsion d'acide linoléique par le fer, méthode modifiée par rapport à celle décrite par Sutherland et col., Arch. Biochem. Biophys., 1982, 214, 1-11.

L'activité dans le choc septique est étudiée chez le rat après induction par une endotoxine lipopolysaccharidique (15 mg/kg), méthode voisine de celle décrite par Terashita et col., Eur. J. Pharmacol., 109, 257-261, 1985.

### Exemples d'effets pharmacologiques

Les composés de formule (1) et leurs sels éventuels augmentent la contraction des veines saphènes animales produite par la noradrénaline et la dépolarisation (solution hyperpotassique) sans affecter, dans la majorité des cas, les réponses contractiles artérielles. A titre illustratif, les composés des exemples 2, 5, 7, 9 (10 nM à 30 mM) augmentent de plus de 40 % (DE50 ± 0,3 microM) les contractions des veines saphènes de lapin produites par le KCl (40 mM).

Les composés des exemples 5, 8, 11 augmentent à leur concentration maximale de 30 à 200 % la contraction des veines saphènes de lapin en réponse à la noradrénaline 0,3 micromolaire.

A titre illustratif, les composés des exemples 1, 18, 16 augmentent la résistance capillaire de base de 10 à 100 %, lorsque celle-ci est mesurée une heure à deux heures après administration de 1-20 mg/kg/i.p. et jusqu'à quatre à six heures après administration orale de 1-20 mg/kg chez le rat.

A titre illustratif, les composés des exemples 15, et 14 diminuent l'hyperperméabilité vasculaire induite par le zymosan lorsque celle-ci est mesurée 2 et 4 heures après administration per os de 1 à 20 mg/kg.

A titre illustratif, les composés des exemples 10 et, 12 administrés deux fois en i.p. à la dose de 5 mg/kg inhibent l'oedème et la migration leucocytaire dans la cavité pleurale, 6 heures après injection de carragénine dans le modèle de la pleurésie chez le rat.

A titre illustratif, les composés des exemples 3 et 1 présentent un effet antiradicalaire dans le modèle utilisant le DPPH.

### Toxicité

Par ailleurs, les composés de formule (1) et leurs sels éventuels sont très peu toxiques. Par exemple, après administration unique per os chez la souris, aucune mortalité n'est observée à la dose de 1 g/kg dans la majorité des cas. Les seuls effets observés sont dans certains cas des diarrhées colorées et des urines colorées, ces dernières étant le témoin d'une résorption du produit.

Ce qui précède montre que les composés de formule (1) et leurs sels éventuels peuvent être utilisés en thérapeutique humaine et animale. Ils sont en particulier indiqués dans l'insuffisance veineuse fonctionnelle, organique et les pathologies hémorroïdaires par leurs composantes vasculaires et antiinflammatoires, ainsi que dans les affections typiquement inflammatoires (ostéoarticulaires, dermatologiques ou cardiovasculaires) et dans les états de chocs constitués par une chute importante de la pression artérielle, en particulier dans les états de chocs septiques (endotoxiques).

L'insuffisance veineuse fonctionnelle se caractérise par une dilatation et une hyperdistensibilité des veines superficielles des membres inférieurs, oedèmes, paresthésies à type d'impatince, de jambe sans repos. Ce type de pathologie peut évoluer vers l'insuffisance veineuse organique (varices, incontinence valvulaire profonde ...) voire vers la phlébothrombose et les lésions ulcéreuses.

Dans cette pathologie veineuse, une composante inflammatoire s'installe dans les premiers stades et se manifeste plus clairement dans les stades avancés.

La présente invention comprend donc l'utilisation des composés de formule (1) ci-dessus décrits et de leurs sels éventuels, comme substances actives pour la préparation de médicaments et compositions pharmaceutiques, à usage humain et vétérinaire, comprenant au moins un desdits composés et sels en association avec un support ou diluant physiologiquement acceptable.

La forme de ces médicaments et compositions pharmaceutiques dépendra bien évidemment de la voie d'administration souhaitée notamment orale, parentérale et rectale et ils peuvent être formulés selon les techniques classiques avec mise en oeuvre des supports et véhicules usuels.

Ainsi, dans le cas d'une administration par voie orale, ils peuvent se présenter sous la forme de comprimés, tablettes, gélules, solutions, sirops et suspensions.

Les comprimés, tablettes et gélules contiennent la substance active conjointement avec un diluant (par exemple lactose, dextrose, sucrose, mannitol, sorbitol ou cellulose), un lubrifiant (par exemple silice, talc ou stéarate comme le stéarate de magnésium), un liant (par exemple amidon, méthylcellulose ou gomme arabique), un agent de désintégration (alginate par exemple) et ils sont fabriqués par des techniques connues par exemple de mélange, de granulation, de pastillage, d'enrobage, etc ...

Les sirops peuvent contenir, à titre de support, glycérol, mannitol et/ou sorbitol. Les solutions et suspensions peuvent comprendre de l'eau et un support tel qu'une gomme naturelle, de la gélose, de l'alginate de sodium ou de l'alcool polyvinylique.

Pour l'administration par voie parentérale, les médicaments et compositions peuvent prendre la forme de solutions, d'émulsions ou de suspensions comprenant la substance active et un support approprié tel que l'eau stérile ou des solutions salines isotoniques aqueuses stériles.

Pour l'administration par voie rectale, ils peuvent prendre la forme de suppositoires comprenant la substance active et un support approprié tel que le beurre de cacao ou du polyéthylène glycol.

La dose thérapeutique des substances actives pourra atteindre 2000 mg/jour suivant la voie d'administration, l'âge, le poids et l'état du sujet à traiter et la puissance thérapeutique de la substance active mise en oeuvre.

## Revendications

1. Dérivés d'indane cétoniques caractérisés en ce qu'ils répondent à la formule : 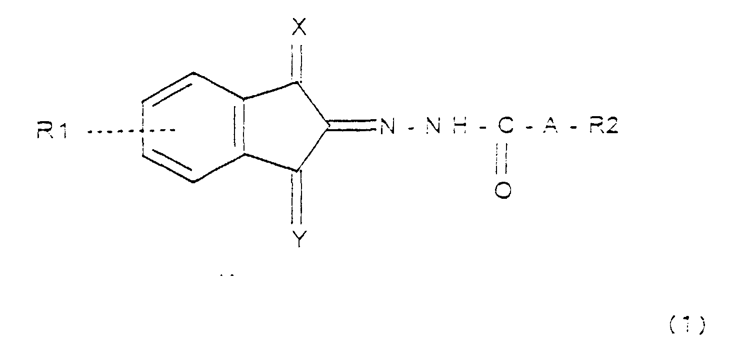 dans laquelle R₁ représente un atome d'hydrogène, d'halogène, un radical de formule NR₃R₃', où, R₃ et R₃' sont des radicaux organiques monovalents en C₁ à C₅ hydrogénés ou oxygénés,
X et Y sont choisis indépendamment parmi l'oxygène, un radical NR₄, ou N₂R₄H, où R₄ est un radical alkyle en C₁ à C₄ ou un radical alcoxylé,
A est un atome d'oxygène, un radical NR₅ où R₅ est un atome d'hydrogène, un radical organique en C₁ à C₅ linéaire ou ramifié,
R₂ est un radical organique monovalent en C₁ à C₆ linéaire ou ramifié ou un cycle aromatique ou hétéroaromatique ayant un ou plusieurs hétéroatomes,
R₂ ayant la définition ci-dessus à l'exclusion du radical phényle ou phényle substitué quand X et Y sont tous deux un oxygène et R₁ est un hydrogène, et à l'exclusion du cas où R₂ est un groupement 4-pyridinyle.

2. Dérivés d'indane cétoniques selon la revendication 1, caractérisés en ce que R₁ est NO₂, X et Y représentent un atome d'oxygène, A est un radical NR₅ où R₅ représente un atome d'hydrogène et R₂ est un radical phényle.

3. Dérivés d'indane cétoniques selon la revendication 1, caractérisés en ce que R₁ est un atome d'hydrogène, X et Y sont équivalents et représentent chacun un atome d'oxygène, A est un radical NR₅ où R₅ représente un atome d'hydrogène et R₂ est un cycle aromatique substitué par un dérivé nitro.

4. Dérivés d'indane cétoniques selon la revendication 1, caractérisés en ce que R₁ représente un atome d'hydrogène, X et Y sont équivalents et représentent un atome d'oxygène, A est un radical NR₅ où R₅ est un atome d'hydrogène, R₂ est un cycle hétéroaromatique ayant un hétéroatome azoté.

5. Dérivés d'indane cétoniques selon la revendication 1, caractérisés en ce que R₁ est un atome d'halogène, X et Y sont équivalents et représentent chacun un atome d'oxygène, A est un radical NR₅ où R₅ représente un atome d'hydrogène et R₂ est un radical phényle.

6. Dérivés d'indane cétoniques selon la revendication 1, caracterisés en ce que R₁ est un atome d'halogène, X et Y sont équivalents et représentent chacun un atome d'oxygène, A est un radical NR₅ où R₅ représente un atome d'hydrogène et R₂ est un cycle aromatique substitué par un atome d'halogène.

7. Dérivés d'indane cétoniques selon la revendication 1, caractérisés en ce que R₁ est un atome d'halogène, X et Y sont équivalents et représentent chacun un atome d'oxygène, A est un radical NR₅ où R₅ est un atome d'hydrogène et R₂ est un cycle hétéroaromatique ayant un hétéroatome azoté.

8. Dérivés d'indane cétoniques selon la revendication 1, caractérisés en ce que R₁ est NO₂, X et Y sont équivalents et représentent un atome d'oxygène, A est une radical NR₅ où R₅ représente un atome d'hydrogène et R₂ est un cycle aromatique substitué par un atome d'halogène.

9. Dérivés d'indane cétoniques selon la revendication 1, caractérisés en ce que R₁ est un radical NO₂, X est analogue à Y et chacun représente un atome d'oxygène, A est un radical NR₅ où R₅ représente un atome d'hydrogène et R₂ est un cycle hétéroaromatique ayant un hétéroatome azoté.

10. Dérivés d'indane cétoniques selon la revendication 1, caractérisés en ce que R₁ représente un atome d'hydrogène, X et Y sont analogues et représentent chacun un atome d'oxygène, A représente un atome d'oxygène et R₂ est un radical tertiobutyle.

11. Dérivés d'indane cétoniques selon la revendication 1, caractérisés en ce que R₁ représente un atome d'hydrogène, X et Y sont analogues et représentent chacun un radical NR₄ où R₄ est un radical méthyle, A représente un atome d'oxygène et R₂ est un radical tertiobutyle.

12. Dérivés d'indane cétoniques selon la revendication 1, caractérisés en ce que R₁ représente un atome d'hydrogène, X et Y sont analogues et représentent chacun un radical NR₄ où R₄ est un radical méthyle, A est un radical NR₅ où R₅ représente un atome d'hydrogène et R₂ est un radical méthyle.

13. Dérivés d'indane cétoniques selon la revendication 1, caractérisés en ce que R₁ représente un atome d'hydrogène, X et Y sont analogues et représentent chacun un radical NR₄ où R₄ est un radical méthyle, A est un radical NR₅ où R₅ représente un atome d'hydrogène et R₂ est un radical phényle.

14. Les sels d'addition d'acides minéraux choisis parmi l'acide chlorhydrique, l'acide bromhydrique, l'acide sulfurique, l'acide phosphorique et l'acide nitrique et d'acides organiques choisis parmi l'acide acétique, l'acide propionique, l'acide oxalique, l'acide citrique, l'acide maléique, l'acide fumarique, l'acide succinique et l'acide tartrique des composés selon l'une quelconque des revendications 1 à 13.

15. Procédé d'obtention de dérivés d'indane cétoniques caractérisé en ce qu'il consiste à faire réagir un composé de formule (I) 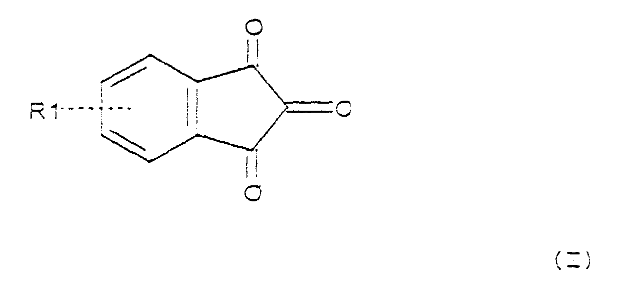 dans laquelle R₁ a la même signification que celle mentionnée dans la revendication 1 avec un composé de formule NH₂-NH-COR₆ où R₆ est choisi parmi les amines substituées ou non substituées, les radicaux alcoxy linéaires ou ramifiés, les radicaux alkyl-amino, aryl-amino substitués ou non substitués pour obtenir un composé de formule (III). 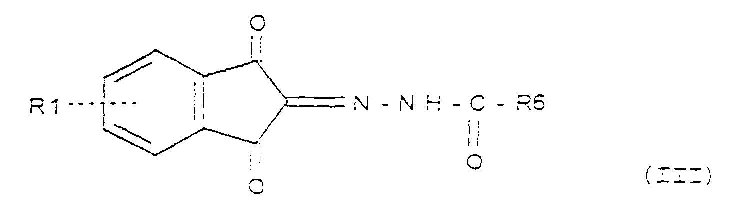 lequel composé de formule (III) est transformé en un composé de formule : 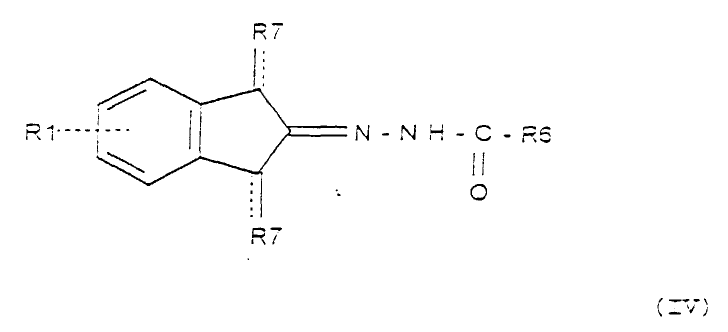 dans laquelle R₇ est un groupement dioxolane ou dioxanne substitué ou non substitué, un radical imine subsitué ou non substitué, un radical hydrazine substitué ou non substitué, à effectuer le clivage du radical COR₆ du composé de formule (IV) pour obtenir le composé de formule : 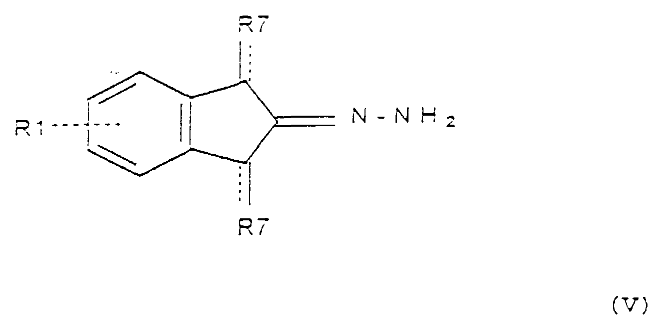 sur lequel on fait réagir un composé de formule : 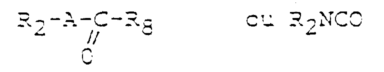 dans laquelle R₂ et A ont la signification mentionnée dans la revendication 1 et R₈ est un radical alcoxy pour obtenir un composé de formule : 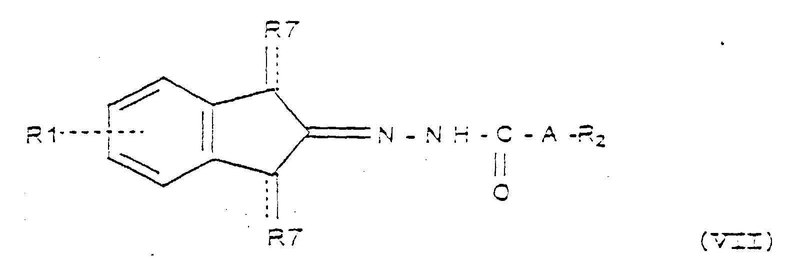 dans laquelle R₁, R₂, R₇ et A ont la même signification que celle indiquée plus haut pour obtenir le composé de formule : 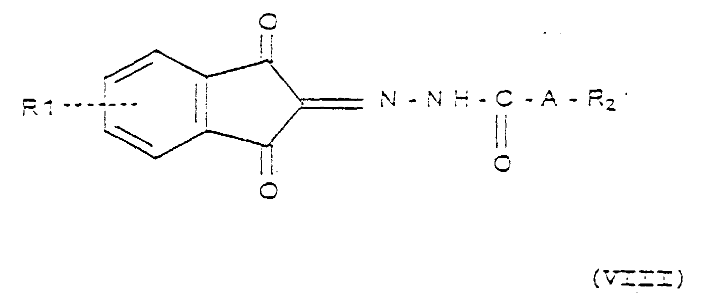 dans laquelle, R₁, A et R₂ ont la même signification que mentionnée ci-dessus.

16. Procédé d'obtention de dérivés d'indane cétoniques selon la revendication 15, caractérisé en ce que le radical R₆ est un radical o-tertiobutyle tandis que le radical R₇ est un radical =NCH₃.

17. Procédé d'obtention de dérivés d'indane cétoniques selon la revendication 16, caractérisé en ce qu'on obtient comme produit intermédiaire le composé de formule 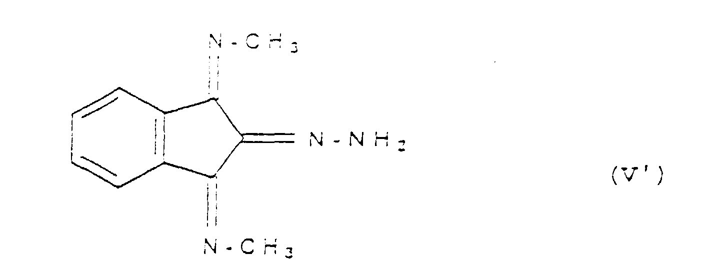 dans laquelle R₇ est =NCH₃.

18. Utilisation des composés selon l'une quelconque des revendications 1 à 14 pour l'obtention d'un médicament destiné au traitement de l'insuffisance veineuse fonctionnelle organique.

19. Utilisation des composés selon l'une quelconque des revendications 1 à 14 pour l'obtention d'un médicament destiné au traitement des pathologies hémorroïdaires.

20. Utilisation des composés selon l'une quelconque des revendications 1 à 14 pour l'obtention d'un médicament destiné au traitement des infections inflammatoires ostéoarticulaires, dermatologiques et cardiovasculaires.

21. Utilisation des composés selon l'une quelconque des revendications 1 à 14 pour l'obtention d'un médicament destiné au traitement des états de chocs constitués par une chute importante de la pression artérielle plus particulièrement dans les états de chocs septiques.

22. A titre de produit intermédiaire la 2-pyridylaminocarbonylhydrazine.

23. A titre de produit intermédiaire la 3-pyridylaminocarbonylhydrazine.

## Patentansprüche

1. Ketonderivate des Indans, dadurch gekennzeichnet, daß sie der folgenden Formel entsprechen: 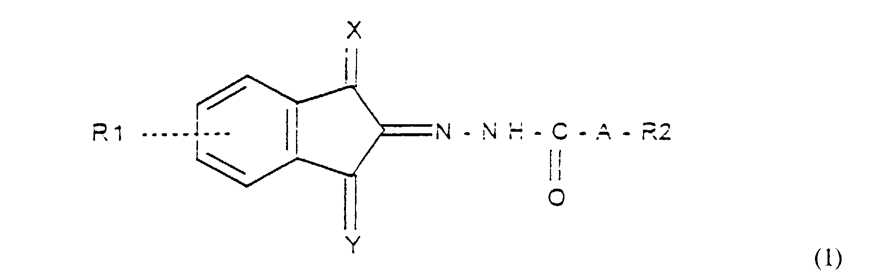 in der R₁ ein Wasserstoffatom, ein Halogenatom, einen Rest mit der Formel NR₃R₃' bedeutet, in dem R₃ und R₃' einwertige organische Reste sind, die an C₁ bis C₅ hydrogeniert oder oxygeniert sind,
X und Y unabhängig voneinander ausgewählt sind aus Sauerstoff, einem NR₄- oder N₂R₄H-Rest, in dem R₄ ein Alkyl-Rest an C₁ bis C₄ oder ein alkoxylierter Rest ist,
A ein Sauerstoffatom, ein NR₅-Rest, in dem R₅ ein Wasserstoffatom ist, ein organischer Rest ist, der an C₁ bis C₅ unverzweigt oder verzweigt ist,
R₂ ein einwertiger organischer Rest ist, der an C₁ bis C₆ unverzweigt oder verzweigt ist, oder ein aromatischer Ring oder ein Heteroaromat mit einem oder mehreren Heteroatomen,
wobei R₂ die vorstehend genannte Definition besitzt mit der Ausnahme des Phenyl-Rests oder eines substituierten Phenyl-Rests, wenn X und Y alle beide ein Sauerstoffatom sind und R₁ ein Wasserstoffatom ist, und mit der Ausnahme des Falles, in dem R₂ eine 4-Pyridinylgruppierung ist.

2. Ketonderivate des Indans gemäß Anspruch 1, dadurch gekennzeichnet, daß R₁ NO₂ ist, X und Y ein Sauerstoffatom bedeuten, A ein NR₅-Rest ist, in dem R₅ ein Wasserstoffatom bedeutet, und R₂ ein Phenyl-Rest ist.

3. Ketonderivate des Indans nach Anspruch 1, dadurch gekennzeichnet, daß R₁ ein Wasserstoffatom ist, X und Y gleich sind und jedes ein Sauerstoffatom bedeutet, A ein NR₅-Rest ist, in dem R₅ ein Wasserstoffatom bedeutet, und R₂ ein aromatischer Ring ist, der mit einem Nitroderivat substituiert ist.

4. Ketonderivate des Indans nach Anspruch 1, dadurch gekennzeichnet, daß R₁ ein Wasserstoffatom bedeutet, X und Y gleich sind und ein Sauerstoffatom bedeuten, A ein NR₅-Rest ist, in dem R₅ ein Wasserstoffatom ist, R₂ ein heteroaromatischer Ring mit einem Stickstoff-Heteroatom ist.

5. Ketonderivate des Indans nach Anspruch 1, dadurch gekennzeichnet, daß R₁ ein Halogenatom ist, X und Y gleich sind und jedes ein Sauerstoffatom bedeutet, A ein NR₅-Rest ist, in dem R₅ ein Wasserstoffatom bedeutet, und R₂ ein Phenyl-Rest ist.

6. Ketonderivate des Indans nach Anspruch 1, dadurch gekennzeichnet, daß R₁ ein Halogenatom ist, X und Y gleich sind und jedes ein Sauerstoffatom bedeutet, A ein NR₅-Rest ist, in dem R₅ ein Wasserstoffatom bedeutet, und R₂ ein aromatischer Ring ist, der mit einem Halogenatom substituiert ist.

7. Ketonderivate des Indans nach Anspruch 1, dadurch gekennzeichnet, daß R₁ ein Halogenatom ist, X und Y gleich sind und jedes ein Sauerstoffatom bedeutet, A ein NR₅-Rest ist, in dem R₅ ein Wasserstoffatom ist, und R₂ ein heteroaromatischer Ring ist mit einem Stickstoff-Heteroatom.

8. Ketonderivate des Indans nach Anspruch 1, dadurch gekennzeichnet, daß R₁ NO₂ ist, X und Y gleich sind und ein Sauerstoffatom bedeuten, A ein NR₅-Rest ist, in dem R₅ ein Wasserstoffatom bedeutet, und R₂ ein aromatischer Ring ist, der mit einem Halogenatom substituiert ist.

9. Ketonderivate des Indans nach Anspruch 1, dadurch gekennzeichnet, daß R₁ ein NO₂-Rest ist, X Y entspricht und jedes ein Sauerstoffatom bedeutet, A ein NR₅-Rest ist, in dem R₅ ein Wasserstoffatom bedeutet, und R₂ ein heteroaromatischer Ring ist mit einem Stickstoff-Heteroatom.

10. Ketonderivate des Indans nach Anspruch 1, dadurch gekennzeichnet, daß R₁ ein Wasserstoffatom bedeutet, X und Y Analoge sind und jedes ein Sauerstoffatom bedeutet, A ein Sauerstoffatom bedeutet und R₂ ein tert.-Butyl-Rest ist.

11. Ketonderivate des Indans nach Anspruch 1, dadurch gekennzeichnet, daß R₁ ein Wasserstoffatom bedeutet, X und Y Analoge sind und jedes einen NR₄-Rest bedeutet, in dem R₄ ein Methyl-Rest ist, A ein Sauerstoffatom bedeutet und R₂ ein tert.-Butyl-Rest ist.

12. Ketonderivate des Indans nach Anspruch 1, dadurch gekennzeichnet, daß R₁ ein Wasserstoffatom bedeutet, X und Y Analoge sind und jedes einen NR₄-Rest bedeutet, in dem R₄ ein Methyl-Rest ist, A ein NR₅-Rest ist, in dem R₅ ein Wasserstoffatom bedeutet, und R₂ ein Methyl-Rest ist.

13. Ketonderivate des Indans nach Anspruch 1, dadurch gekennzeichnet, daß R₁ ein Wasserstoffatom bedeutet, X und Y Analoge sind und jedes einen NR₄-Rest bedeutet, in dem R₄ ein Methyl-Rest ist, A ein NR₅-Rest ist, in dem R₅ ein Wasserstoffatom bedeutet, und R₂ ein Phenyl-Rest ist.

14. Additionssalze von Mineralsäuren ausgewählt aus Salzsäure, Bromwasserstoffsäure, Schwefelsäure, Phosphorsäure und Salpetersäure und organischen Säuren ausgewählt aus Essigsäure, Propionsäure, Oxalsäure, Zitronensäure, Maleinsäure, Fumarsäure, Bernsteinsäure und Weinsäure, von Verbindungen nach einem der Ansprüche 1 bis 13.

15. Verfahren zur Herstellung von Ketonderivaten des Indans, dadurch gekennzeichnet, daß es darin besteht, daß eine Verbindung der Formel (I) 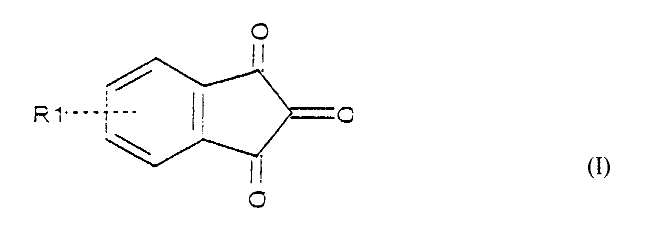 in der R₁ die gleiche Bedeutung besitzt, wie die in Anspruch 1 erwähnte, mit einer Verbindung der Formel NH₂-NH-COR₆ umgesetzt wird, in der R₆ ausgewählt ist aus substituierten und unsubstituierten Aminen, unverzweigten und verzweigten Alkoxy-Resten, Alkylamino-Resten und substituierten oder unsubstituierten Arylamino-Resten, zum Erhalten einer Verbindung der Formel (III): 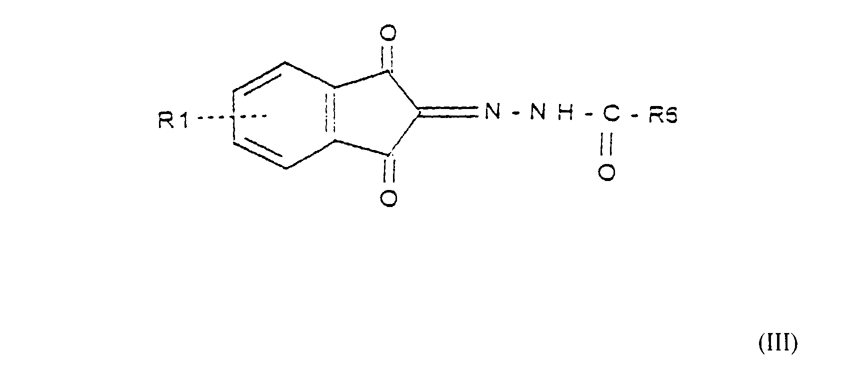 die Verbindung der Formel (III) in eine Verbindung der folgenden Formel überführt wird: 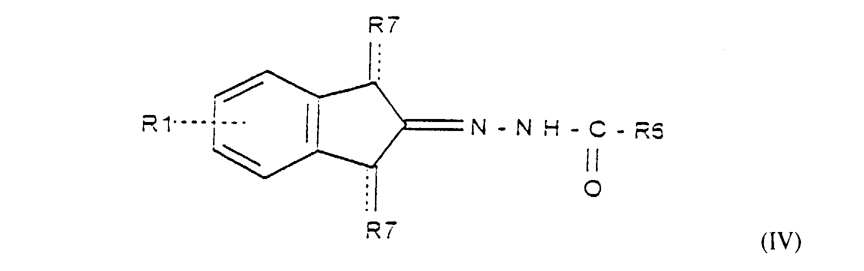 in der R₇ eine substituierte oder unsubstituierte Dioxolan- oder Dioxan-Gruppierung ist, ein substituierter oder unsubstituierter Imin-Rest, ein substituierter oder unsubstituierter Hydrazin-Rest, zum Bewirken der Spaltung des COR₆-Rests der Verbindung der Formel (IV) zum Erhalten der Verbindung der folgenden Formel: 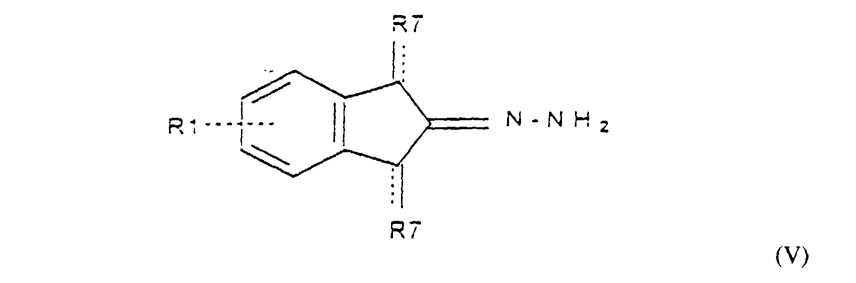 mit der man eine Verbindung der folgenden Formel umsetzt: 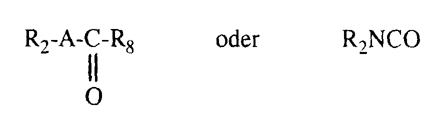 in der R₂ und A die in Anspruch 1 erwähnte Bedeutung besitzen und R₈ ein Alkoxy-Rest ist, zum Erhalten einer Verbindung der folgenden Formel: 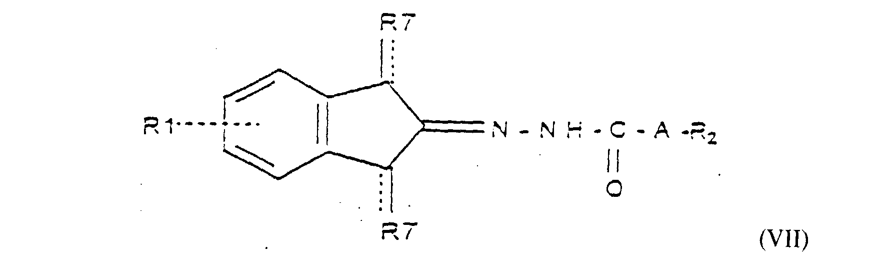 in der R₁, R₂, R₇ und A die gleiche Bedeutung wie die weiter vorstehend angegebene besitzen, zum Erhalten der Verbindung der folgenden Formel: 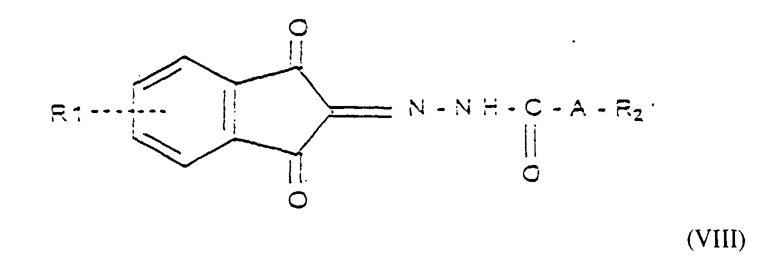 in der R₁, A und R₂ die gleiche Bedeutung besitzen, wie vorstehend erwähnt.

16. Verfahren zur Herstellung von Ketonderivaten des Indans nach Anspruch 15, dadurch gekennzeichnet, daß R₆ ein o-tert.-Butyl-Rest ist, während der Rest R₇ ein =NCH₃-Rest ist.

17. Verfahren zur Herstellung von Ketonderivaten des Indans nach Anspruch 16, dadurch gekennzeichnet, daß man als Zwischenprodukt die Verbindung der folgenden Formel erhält: 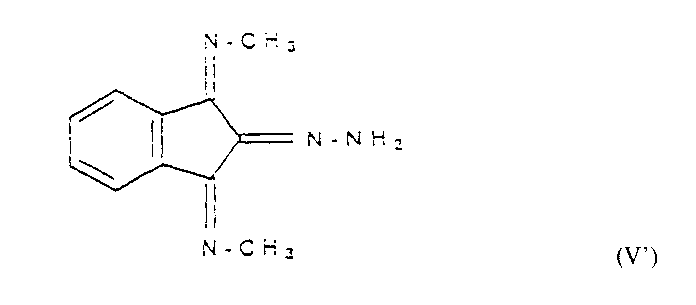 in der R₇ =NCH₃ ist.

18. Verwendung der Verbindungen nach einem der Ansprüche 1 bis 14 zur Herstellung eines Medikaments zur Behandlung einer funktionellen organischen Veneninsuffizienz.

19. Verwendung der Verbindungen nach einem der Ansprüche 1 bis 14 zur Herstellung eines Medikaments zur Behandlung von hämorrhoidalen Krankheiten.

20. Verwendung der Verbindungen nach einem der Ansprüche 1 bis 14 zur Herstellung eines Medikaments zur Behandlung von entzündlichen osteoartikulären, dermatologischen und kardiovaskulären Infektionen.

21. Verwendung der Verbindungen nach einem der Ansprüche 1 bis 14 zur Herstellung eines Medikaments zur Behandlung von Schockzuständen, die durch einen bedeutenden arteriellen Druckabfall, insbesondere bei septischen Schockzuständen, gebildet werden.

22. Die Verbindung 2-Pyridylaminocarbonylhydrazin als Zwischenprodukt.

23. Die Verbindung 3-Pyridylaminocarbonylhydrazin als Zwischenprodukt.

## Claims

1. Ketone derivatives of indane, characterized in that they have the following formula: 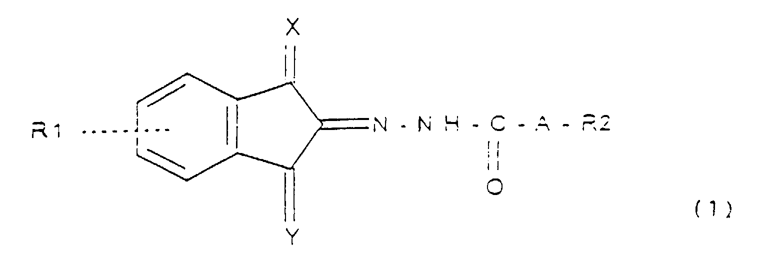 where R₁ represents a hydrogen or halogen atom, or a radical with formula NR₃R₃', where R₃ and R₃' are monovalent hydrogen-containing or oxygen-containing C₁ to C₅ organic radicals;
X and Y are independently selected from oxygen, an NR₄ or N₂R₄H radical, where R₄ is a C₁ to C₄ alkyl radical, or an alkoxylated radical;
A is an oxygen atom, an NR₅ radical where R₅ is a hydrogen atom, or a linear or branched C₁ to C₅ organic radical;
R₂ is a monovalent linear or branched C₁ to C₆ organic radical or an aromatic or heteroaromatic cycle containing one or more heteroatoms;
R₂ having the above definition excluding a phenyl or substituted phenyl radical when X and Y are both oxygen and R₁ is hydrogen, and excluding the case where R₂ is a 4-pyridinyl group.

2. Ketone derivatives of indane according to claim 1, characterized in that R₁ is NO₂, X and Y represent an oxygen atom, A is an NR₅ radical where R₅ represents a hydrogen atom, and R₂ is a phenyl radical.

3. Ketone derivatives of indane according to claim 1, characterized in that R₁ is a hydrogen atom, X and Y are equivalent and each represent an oxygen atom, A is an NR₅ radical where R₅ represents a hydrogen atom, and R₂ is an aromatic cycle substituted by a nitro derivative.

4. Ketone derivatives of indane according to claim 1, characterized in that R₁ represents a hydrogen atom, X and Y are equivalent and represent an oxygen atom, A is an NR₅ radical where R₅ represents a hydrogen atom, and R₂ is a heteroaromatic cycle containing a nitrogen heteroatom.

5. Ketone derivatives of indane according to claim 1, characterized in that R₁ is a halogen atom, X and Y are equivalent and each represent an oxygen atom, A is an NR₅ radical where R₅ represents a hydrogen atom, and R₂ is a phenyl radical.

6. Ketone derivatives of indane according to claim 1, characterized in that R₁ is a halogen atom, X and Y are equivalent and each represent an oxygen atom, A is an NR₅ radical where R₅ represents a hydrogen atom, and R₂ is a an aromatic cycle substituted by a halogen atom.

7. Ketone derivatives of indane according to claim 1, characterized in that R₁ is a halogen atom, X and Y are equivalent and each represent an oxygen atom, A is an NR₅ radical where R₅ is a hydrogen atom, and R₂ is a heteroaromatic cycle containing a nitrogen heteroatom.

8. Ketone derivatives of indane according to claim 1, characterized in that R₁ is NO₂, X and Y are equivalent and represent an oxygen atom, A is an NR₅ radical where R₅ represents a hydrogen atom, and R₂ is an aromatic cycle substituted by a halogen atom.

9. Ketone derivatives of indane according to claim 1, characterized in that R₁ is an NO₂ radical, X is analogous to Y and each represents an oxygen atom, A is an NR₅ radical where R₅ represents a hydrogen atom, and R₂ is a heteroaromatic cycle containing a nitrogen heteroatom.

10. Ketone derivatives of indane according to claim 1, characterized in that R₁ represents a hydrogen atom, X and Y are analogous and each represent an oxygen atom, A represents an oxygen atom and R₂ is a tertiobutyl radical.

11. Ketone derivatives of indane according to claim 1, characterized in that R₁ represents a hydrogen atom, X and Y are analogous and each represent an NR₄ radical where R₄ is a methyl radical, A represents an oxygen atom and R₂ is a tertiobutyl radical.

12. Ketone derivatives of indane according to claim 1, characterized in that R₁ represents a hydrogen atom, X and Y are analogous and each represent an NR₄ radical where R₄ is a methyl radical, A is an NR₅ radical where R₅ represents a hydrogen atom, and R₂ is a methyl radical.

13. Ketone derivatives of indane according to claim 1, characterized in that R₁ represents a hydrogen atom, X and Y are analogous and each represent an NR₄ radical where R₄ is a methyl radical, A is an NR₅ radical where R₅ represents a hydrogen atom, and R₂ is a phenyl radical.

14. Addition salts of mineral acids selected from hydrochloric acid, hydrobromic acid, sulphuric acid, phosphoric acid and nitric acid, and of organic acids selected from acetic acid, propionic acid, oxalic acid, citric acid, maleic acid, fumaric acid, succinic acid and tartaric acid with compounds according to any one of claims 1 to 13.

15. A process for producing ketone derivatives of indane, characterized in that it consists of reacting a compound with formula (I) 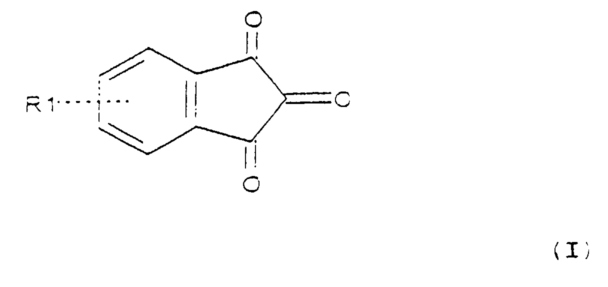 where R₁ has the meaning defined in claim 1, with a compound with formula NH₂-NH-COR₆ where R₆ is selected from substituted or non substituted amines, linear or branched alkoxy radicals, alkylamino radicals, and substituted or non substituted arylamino radicals, to obtain a compound with formula (III): 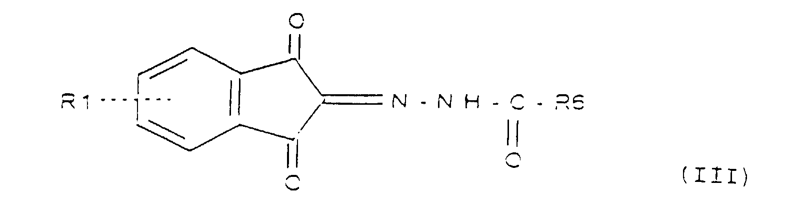 then transforming said compound with formula (III) into a compound with formula: 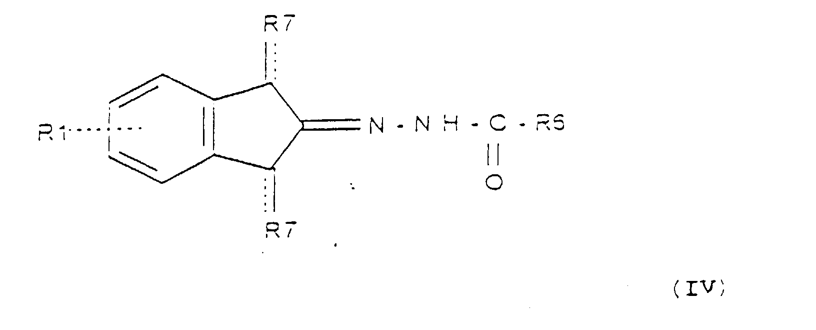 where R₇ is a substituted or non substituted dioxolane or dioxane group, a substituted or non substituted imine, or a substituted or non substituted hydrazine radical, then cleaving the COR₆ radical from the compound with formula (IV) to obtain a compound with formula: 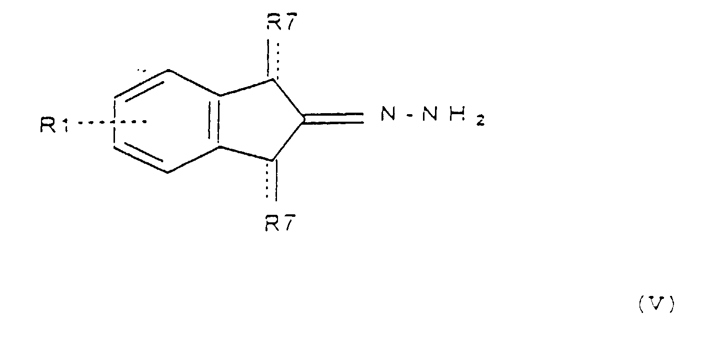 then reacting it with a compound with formula: 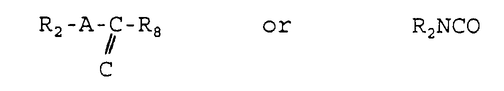 where R₂ and A have the meanings defined in claim 1 and R₈ is an alkoxy radical, to obtain a compound with formula: 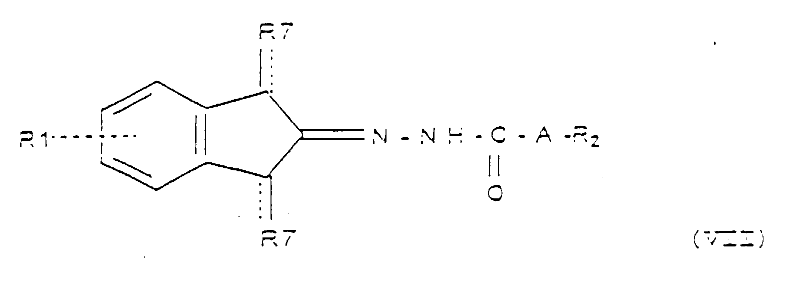 where R₁, R₂, R₇ and A have the meanings defined above, to obtain a compound with formula: 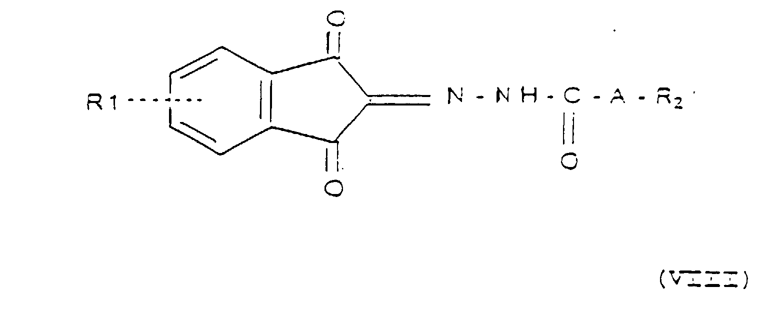 where R₁, A and R₂ have the meanings given above.

16. A process for producing ketone derivatives of indane according to claim 15, characterized in that radical R₆ is an o-tertiobutyl radical while radical R₇ is a =NCH₃ radical.

17. A process for producing ketone derivatives of indane according to claim 16, characterized in that a compound with the following formula is obtained as an intermediate compound: 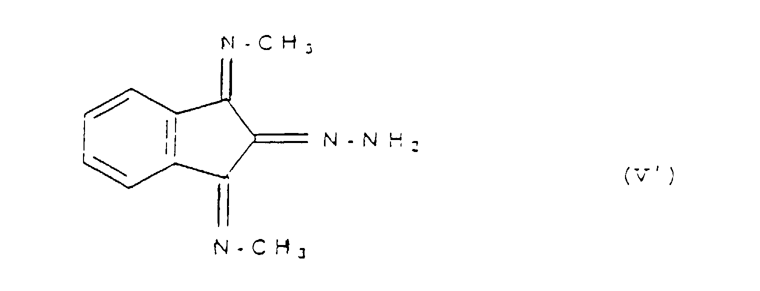 where R₇ is =NCH₃.

18. Use of compounds according to any one of claims 1 to 14 to obtain a drug intended for treatment of functional organic venous insufficiency.

19. Use of compounds according to any one of claims 1 to 14 to obtain a drug intended for the treatment of hemorrhoidal pathologies.

20. Use of compounds according to any one of claims 1 to 14 to obtain a drug intended for treatment of osteoarticulatory, dermatological and cardiovascular inflammatory infections.

21. Use of compounds according to any one of claims 1 to 14 to obtain a drug intended for treatment of shock constituted by a substantial drop in blood pressure, more particularly for septic shock.

22. 2-pyridyl-aminocarbonylhydrazine as an intermediate product.

23. 3-pyridyl-aminocarbonylhydrazine as an intermediate product.
